# EUROPÄISCHE PATENTSCHRIFT

(11) **EP 0 388 838 B1**
(45) Veröffentlichungstag und Bekanntmachung des Hinweises auf die Patenterteilung: **10.01.1996**
(21) Anmeldenummer: 90105116.9
(22) Anmeldetag: 19.03.1990
(51) Int. Cl.: C07D 239/42, C07D 239/46, C07D 239/48, A01N 43/54

(54) **Schädlingsbekämpfungsmittel**
Parasiticide
Parasiticide

(30) Priorität: 22.03.1989 CH 1073/89; 17.10.1989 CH 3771/89
(43) Veröffentlichungstag der Anmeldung: 26.09.1990
(73) Patentinhaber: CIBA-GEIGY AG, CH-4002 Basel (CH)
(72) Erfinder: Hubele, Adolf, Dr., CH-4312 Magden (CH)
(74) Vertreter: Zumstein, Fritz, Dr.

(56) Entgegenhaltungen:
- EP-A- 0 135 472
- EP-A- 0 295 210
- EP-A- 0 337 943

## Beschreibung

Die vorliegende Erfindung betrifft 2-Anilino-pyrimidin-Derivate der nachstehenden Formel I, die Herstellung dieser Stoffe sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem von pflanzenschädigenden Mikroorganismen, vorzugsweise Pilzen.

Die erfindungsgemässen Verbindungen entsprechen der allgemeinen Formel I 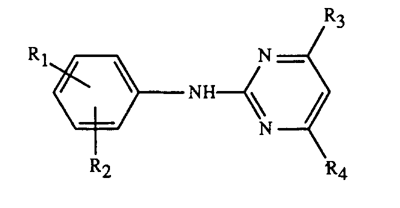
in welcher bedeuten: R₁ und R₂ unabhängig voneinander Wasserstoff, Halogen, C₁-C₃-Alkyl, C₁-C₂-Halogenalkyl, C₁-C₃-Alkoxy oder C₁-C₃-Halogenalkoxy;
R₃ C₃-C₆-Cycloalkyl oder durch Methyl oder Halogen bis zu dreifach gleich oder verschieden substituiertes C₃-C₆-Cycloalkyl;
R₄ Halogen, Thiocyano(-SCN), -OR₅, -SR₅, -NR₅R₆, wobei
R₅
a) - Wasserstoff, unsubstituiertes C₁-C₈-Alkyl, oder aber eine C₁-C₄-Alkylgruppe darstellt, die durch Halogen, Hydroxy, Cyano, C₁-C₄-Alkoxy, C₁-C₄-Alkylamino, Bis(C₁-C₄-Alkyl)amino, C₃-C₆-Cycloalkyl, oder durch substituiertes oder unsubstituiertes Phenyl oder durch -CO-OC₁-C₃-Alkyl substituiert ist; oder
b) - unsubstituiertes oder durch Methyl substituiertes C₃-C₆-Cycloalkyl darstellt; oder
c) - unsubstituiertes oder durch Halogen substituiertes C₃-C₆-Alkenyl darstellt; oder
d) - unsubstituiertes oder durch Halogen substituiertes C₃-C₆-Alkinyl darstellt; oder
e) - unsubstituiertes oder durch Halogen, C₁-C₃-Alkyl, C₁-C₃-Alkoxy, Nitro substituiertes Phenyl darstellt; oder
f) - einen unsubstituierten oder durch Halogen, C₁-C₃-Alkyl, C₁-C₃-Alkoxy, Nitro substituierten 5-gliedrigen oder 6-gliedrigen gegebenenfalls über -CH₂-gebundenen heterocyclischen Rest mit ein bis drei Heteroatomen N, O oder S darstellt; oder
g) - einen Acylrest -CO-R' darstellt, wobei R' unsubstituiertes oder durch Halogen, C₁-C₃-Alkoxy substituiertes C₁-C₆-Alkyl; unsubstituiertes oder durch Halogen substituiertes C₃-C₆-Alkenyl; unsubstituiertes oder durch Halogen, C₁-C₃-Alkyl, C₁-C₃-Alkoxy, Nitro substituiertes Phenyl bedeutet; oder
h) - einen Carbamoylrest -CO-NH-R'' oder einen Oxycarbonylrest -CO-OR'' darstellt, in welchem R'' einen aliphatischen oder cycloaliphatischen Rest mit maximal 6 C-Atomen bedeutet, der unsubstituiert oder halogensubstituiert ist, oder worin R'' einen Phenyl- oder Benzylrest bedeutet, der je unsubstituiert oder im aromatischen Ring durch Halogen, C₁-C₃-Alkyl, C₁-C₃-Alkoxy, Nitro substituiert ist; und worin
R₆ Wasserstoff, unsubstituiertes C₁-C₈-Alkyl oder aber C₁-C₄-Alkyl darstellt, das durch Halogen, Hydroxy, Cyano, C₁-C₄-Alkoxy substituiert ist; oder worin R₆ unsubstituiertes oder durch Methyl substituiertes C₃-C₆-Cycloalkyl; je unsubstituiertes oder durch Halogen substituiertes C₃-C₆-Alkenyl oder C₃-C₆-Alkinyl; oder unsubstituiertes oder durch Halogen, C₁-C₃-Alkyl, C₁-C₃-Alkoxy, Nitro substituiertes Phenyl darstellt; wobei im Falle R₄ = NR₅R₆ die Substituenten R₅ und R₆ zusammen mit dem N-Atom auch gemeinsam einen Aziridinring oder einen 5- oder 6-gliedrigen heterocyclischen Rest bilden können, der zusätzlich zu dem N-Atom noch einen oder zwei Heteroatome H, O oder S enthalten und alkylsubstituiert sein kann.

Unter Alkyl selbst oder unter Alkyl als Bestandteil eines anderen Substituenten, wie Halogenalkyl, Alkoxy oder Halogenalkoxy, ist je nach Anzahl der benannten Kohlenstoffatome beispielsweise Methyl, Ethyl, Propyl, Butyl, Pentyl, Hexyl, Heptyl, Octyl, sowie ihre Isomeren, wie z.B. Isopropyl, Isobutyl, Isoamyl, tert.-Butyl oder sek.-Butyl zu verstehen.

Unter einem aliphatischen Rest wird wahlweise eine Alkyl-, Alkenyl- oder Alkinylgruppe verstanden. Entsprechend wird unter einem cycloaliphatischen Rest ein gesättigter oder ungesättigter cyclischer Kohlenwasserstoffrest verstanden.

C₃-C₆-Alkenyl stellt einen aliphatischen Rest mit einer Doppelbindung dar, z.B. Allyl, Methallyl, Crotyl, Butenyl, Pentenyl usw.

Ein C₃-C₆-Alkinylrest enthält eine Dreifachbindung, wie z.B. Propargyl, Propin-1-yl, Butinyl usw.

Halogen steht für Fluor, Chlor, Brom oder Jod.

Halogenalkyl und Halogenalkoxy bezeichnen einfach bis perhalogenierte Reste, wie z.B. CHCl₂, CH₂F, CCl₃, CH₂Cl, CHF₂, CF₃, CH₂CH₂Br, C₂Cl₅, CHBr, CHBrCl usw., vorzugsweise CF₃ und CHF₂.

Cycloalkyl steht je nach Zahl der genannten Kohlenstoffatome z.B. für Cyclopropyl, Cyclobutyl, Cyclopentyl oder Cyclohexyl.

In einem substituierten Phenylring können 1 bis 5, vorzugsweise 1 bis 3, gleiche oder verschiedene Substituenten auftreten, ausgewählt aus Halogen, C₁-C₃-Alkyl, C₁-C₃-Alkoxy und Nitro, wobei es für den Fachmann selbstverständlich ist, dass ein zum Zerfall neigender Substituent die Nitro und dass räumlich sperrige Substituenten wie Isopropyl oder Jod höchstens dreimal vorhanden sein können.

Beispiele für Heterocyclen der Gruppe -NR₅R₆ sind Imidazol, Oxazol, Thiazol, 1,2,4-Triazol, Pyrrol, Pyrrolidin, Piperidin, Morpholin, Thiomorpholin, 2,6-Dimethylmorpholin u.a.

Weitere Beispiele gemäss Definition für R₅ allein sind die vorgenannten, auch über Kohlenstoff gebundenen Heterocyclen sowie ferner auch Furan, Tetrahydrofuran (= THF), Thiophen, Pyridin, Picolin, Pyrazin, Triazin, Oxazin, Dioxan u.a.

N-Pyrimidinylanilinverbindungen sind bereits bekannt. So sind in der EP-A-337943 2-Anilinopyrimidinverbindungen als Zwischenprodukte für die Herstellung in N-Phenyl-N-pyrimidinyl-Harnstoffen aufgeführt, die herbizide Eigenschaften aufweisen. In der EP-A-135472, EP-A-295210, EP-A-0 224 339 und der DD-Patentschrift 151 404 werden Verbindungen, die eine N-2-Pyrimidinylstruktur aufweisen, als wirksam gegen pflanzenschädigende Fungi beschrieben. Die bekannten Verbindungen konnten jedoch bisher die in der Praxis an sie gestellten Forderungen nicht befriedigen. Die erfindungsgemässen Verbindungen der Formel I unterscheiden sich in charakteristischer Weise von den bekannten Verbindungen durch die Einführung spezifischer Substituenten R₃ und R₄ und deren Kombination in die Anilinopyrimidin-Struktur, wodurch bei den neuen Verbindungen eine unerwartet hohe mikrobizide Wirksamkeit erreicht wird.

Die Verbindungen der Formel I sind bei Raumtemperatur stabile Oele, Harze oder Feststoffe. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende fungizide Wirkung, sondern auch durch besonders gute Pflanzenverträglichkeit aus.

Eine besondere Gruppe stellen folgende Verbindungen der Formel I dar, in denen R₁ und R₂ Wasserstoff bedeutet; und
R₃ C₃-C₄-Cycloalkyl, das unsubstituiert oder durch Methyl oder Halogen monosubstituiert ist;
R₄ Halogen, -OR₅, -NR₅R₆ bedeuten, wobei
R₅ die Bedeutungen a), b), c), d) oder g) hat, und
R₆ Wasserstoff oder einen Alkyl-, Alkenyl- oder Alkinylrest darstellt, der wahlweise unsubstituiert oder halogensubstituiert ist; oder wobei
R₅ und R₆ zusammen mit dem gemeinsamen N-Atom einen Aziridinring, oder einen wahlweise unsubstituierten oder methylsubstituierten Piperidin-oder Morpholinring darstellen (= Verbindungsgruppe Ib).

Eine weitere wichtige Gruppe sind jene Verbindungen der Formel I, worin bedeuten: R₁ und R₂ Wasserstoff;
R₃ Cyclopropyl oder durch Methyl oder Halogen ein- oder zweifach gleich oder verschieden substituiertes Cyclopropyl;
R₄ Halogen, -SR₅, -OR₅, -NR₅R₆; und
R₅ Wasserstoff oder eine C₁-C₄-Alkylgruppe darstellt, die unsubstituiert oder durch Halogen, Hydroxy, C₁-C₄-Alkoxy, C₁-C₄-Alkylamino, Bis(C₁-C₄-Alkyl)amino, Cyclopropyl oder durch -COOC₁-C₃-Alkyl substituiert ist; oder worin R₅ C₃-C₄-Alkenyl oder C₃-C₄-Alkinyl darstellt oder eine unsubstituierte oder durch Halogen, Methyl, oder Methoxy substituierte Phenylgruppe oder einen Acylrest gemäss der Bedeutung g) darstellt; und R₆ Wasserstoff oder eine C₁-C₄-Alkylgruppe darstellt, die unsubstituiert oder durch Halogen, Hydroxy, C₁-C₄-Alkoxy, C₁-C₄-Alkylamino oder Bis(C₁-C₄-Alkyl)amino substituiert ist oder worin R₆ C₃-C₄-Alkenyl oder C₃-C₄-Alkinyl darstellt (= Verbindungsgruppe Ic).

Unter diesen Verbindungen der Formel Ic sind solche bevorzugt, bei denen
R₁ und R₂ Wasserstoff bedeutet,
R₃ Cyclopropyl darstellt,
R₄ Halogen, -OR₅ oder -NR₅R₆ ist, und
R₅ Wasserstoff oder R₆ bedeutet, und
R₆ C₁-C₃-Alkyl darstellt, das unsubstituiert oder durch C₁-C₄-Alkoxy oder Cyclopropyl monosubstituiert oder durch Halogen bis zu fünffach substituiert ist; oder C₃-C₄-Alkenyl oder Alkinyl bedeutet (= Verbindungsgruppe Id).

Eine weitere bevorzugte Gruppe stellen folgende Verbindungen der Formel I dar, in denen R₁ und R₂ Wasserstoff bedeutet; und
R₃ C₃-C₄-Cycloalkyl, das unsubstituiert oder durch Methyl oder Halogen monosubstituiert ist;
R₄ Halogen bedeutet (Verbindungsgruppe Ie).

Die Verbindungen der Formel I werden hergestellt, indem man
1a) ein Phenylguanidinsalz der Formel IIa 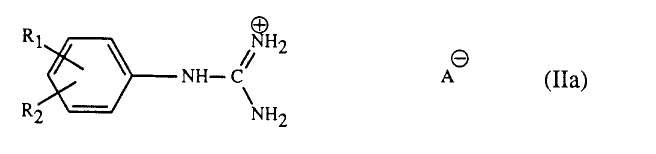 oder das zugrundeliegende Guanidin der Formel IIb 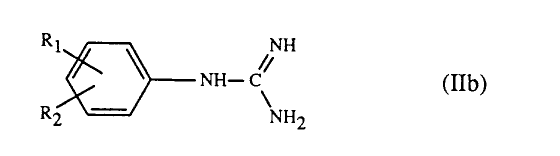 mit einem β-Ketoester der Formel III, 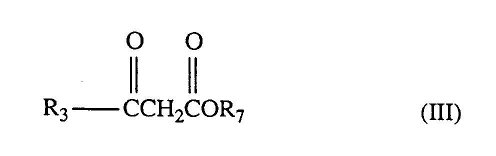 worin R₁, R₂, R₃ die für Formel I gegebene Bedeutung haben und R₇ C₃-C₆-Alkenyl, C₃-C₆-Alkinyl, unsubstituiertes oder mit Halogen und/oder C₁-C₃-Alkyl substituiertes Benzyl oder C₁-C₈-Alkyl bedeuten, ohne Lösungsmittel oder in einem Lösungsmittel, bevorzugt in einem protischen Lösungsmittel, bei Temperaturen von 60°C bis 160°C, bevorzugt 60°C bis 110°C, zu der Verbindung Ia umsetzt: 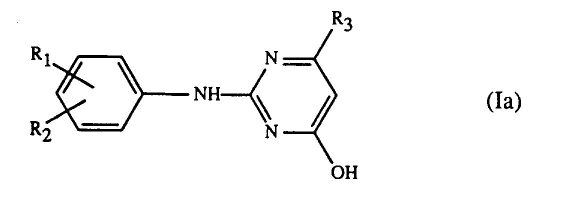 und, sofern gewünscht, die OH-Gruppe gegen Halogen austauscht und zu Verbindungen der Formel Ib gelangt 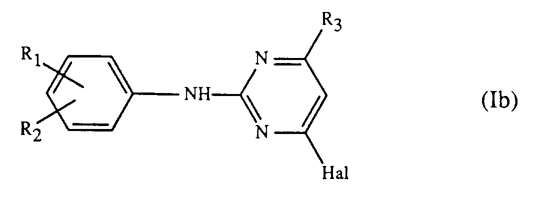 und, sofern gewünscht, weiteren Austausch des Halogenatoms gegen eine der übrigen für R₄ genannten Gruppen, wie weiter unten erläutert.
1b) Zu der Verbindung Ia gelangt man nach einer weiteren Verfahrensvariante auch in einem mehrstufigen Verfahren, indem ein Isothiuroniumsalz der Formel IV 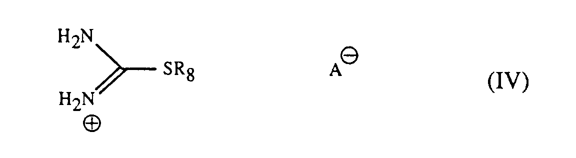 wobei A^{⊖} ein beliebiges Anion wie Chlorid, Sulfat, Phosphat und dgl. und R₈ C₁-C₈-Alkyl oder unsubstituiertes oder mit Halogen und/oder C₁-C₄-Alkyl substituiertes Benzyl bedeuten, mit einem β-Ketoester der Formel III, bevorzugt in einem Lösungsmittel, bei Temperaturen von 40°C bis 140°C, bevorzugt bei 60°C bis 100°C zur Reaktion bringt, wobei sich das Thiopyrimidin V bildet. 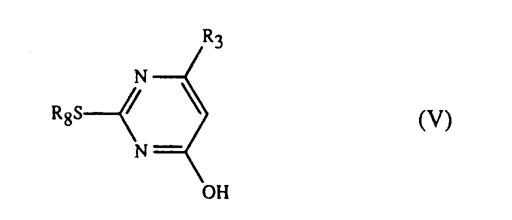 Die so erhaltene Verbindung der Formel V wird mit einem Oxydationsmittel, z.B. mit einer Persäure, zu der Pyrimidinverbindung der Formel VI 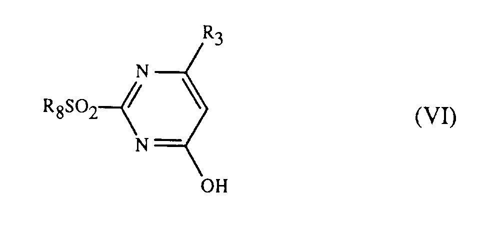 oxydiert und diese mit einem Formylanilin der Formel VII 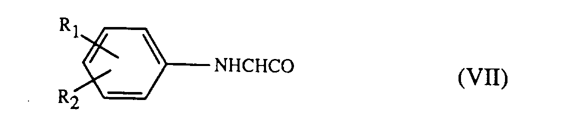 in einem inerten Lösungsmittel in Gegenwart einer Base als Protonenakzeptor bei Temperaturen zwischen -30°C bis +120°C zu einer Verbindung der Formel VIII 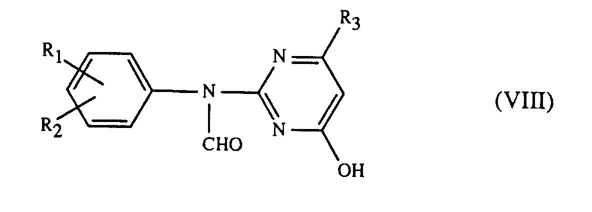 umgesetzt. Die erhaltene Verbindung VIII wird einer Hydrolyse in Gegenwart einer Base, z.B. Alkalihydroxid, oder einer Säure, z.B. Halogenwasserstoffsäure oder Schwefelsäure, in Wasser oder wässrigen Lösungsmittelgemischen, wie wässrigen Alkoholen oder Dimethylformamid, bei Temperaturen von 10°C bis 110°C, bevorzugt 30°C bis 60°C, unterworfen und liefert dann die Verbindung der Formel Ia.

Zu der Verbindung Ib gelangt man, wenn in Verbindung Ia Hydroxyl durch Halogen ersetzt wird: 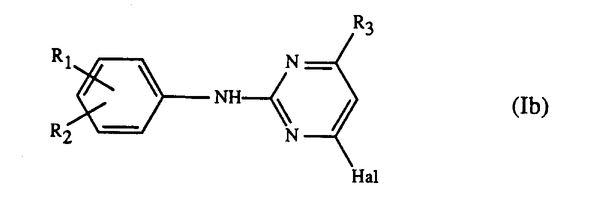
Dies geschieht mit Halogenverbindungen des drei- oder fünfwertigen Phosphors in Gegenwart oder Abwesenheit eines Protonenakzeptors wie z.B. Diethylanilin, wobei die Phosphoroxyhalogenide bevorzugt eingesetzt werden. Die Umsetzungen werden in einem inerten Lösungsmittel oder ohne Lösungsmittel bei Temperaturen zwischen 60°C bis 140°C, bevorzugt 70°C bis 120°C, durchgeführt.

Zu den übrigen Verbindungen der Formel I, in der R₄ eine andere Bedeutung als -OH oder Halogen hat, gelangt man durch Umsetzung einer Verbindung der Formel Ib mit einer Verbindung

HOR₅ (IX)

HSR₅ (X)

H₂NR₅ (XI)

HNR₅R₆ (XII)

Diese Umsetzungen können in einem inerten Lösungsmittel durchgeführt werden. Sind die Reaktanden Alkohole, so können diese als Lösungsmittel verwendet werden. Bei den Verbindungen IX und X sind Protonenakzeptoren nötig, wozu z.B. Alkali- oder Erdalkalihydroxide, das Alkali- oder Erdalkalisalz von IX oder X, oder Metallhydride dienen.

Bei der Verwendung von XI oder XII kann als Protonenakzeptor ausserdem ein Ueberschuss an dem entsprechenden Amin verwendet werden.

Die Reaktionen werden hierbei bei -30°C bis +140°C, bevorzugt bei -10°C bis +110°C durchgeführt.

Ist in der Formel I 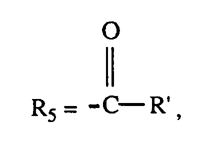
so werden bevorzugt für die Umsetzungen Säurehalogenide XIII 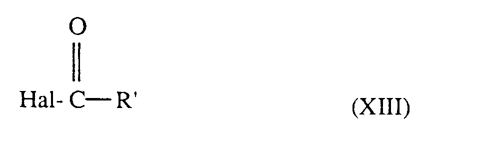
oder Säureanhydride XIV verwendet. 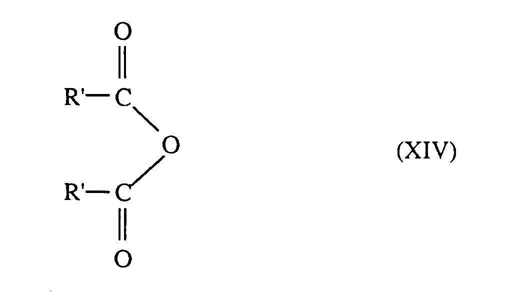
Ist in der Formel I 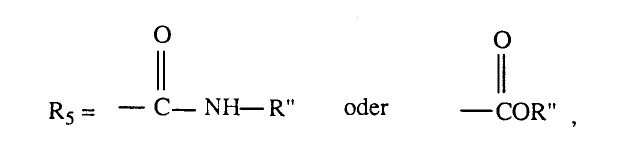
so gelangen entweder Isocyanate XV 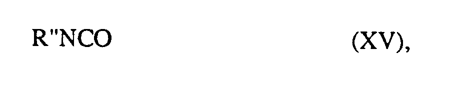
Ameisensäurehalogenide XVI oder Ameisensäureanhydride XVII zur Reaktion: 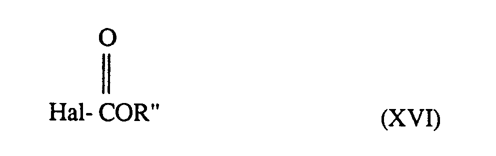 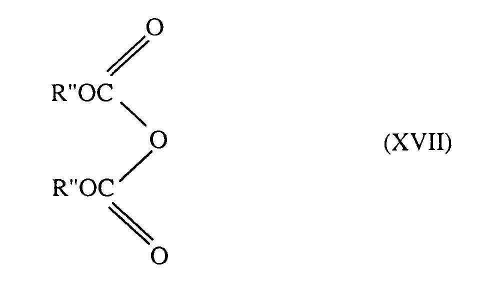
Die beschriebenen Herstellungsverfahren einschliesslich aller Teilschritte sind ein Bestandteil der vorliegenden Erfindung.

Ueberraschenderweise wurde nun gefunden, dass Verbindungen der Formel I ein für praktische Bedürfnisse sehr günstiges biozides Spektrum zur Bekämpfung von Insekten und phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfruchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zietpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl oder -ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden. Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

### Herstellungsbeispiele

### Beispiel 1: Herstellung von 2-Phenylamino-4-hydroxy-6-cyclopropyl-pyrimidin der Formel

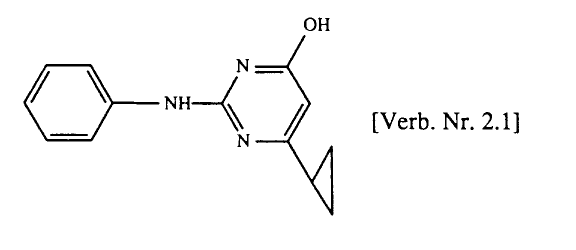

19,7 g (0,1 Mol) Phenylguanidin-hydrogencarbonat und 15,6 g (0,1 Mol) 3-Cyclopropyl-3-oxopropionsäureethylester in 100 ml Ethanol werden unter Rühren 16 Stunden unter Rückfluss erhitzt, wobei sich unter Kohlendioxidbildung eine rötlich gefärbte Lösung bildet. Nach dem Abkühlen auf Raumtemperatur wird mit Aktivkohle behandelt, filtriert und das Filtrat mit 400 ml Wasser versetzt. Das ausgeschiedene graue Pulver wird säulenchromatographisch über Kieselgel (Methanol/Chloroform 1:1) gereinigt. Nach dem Abdampfen des Laufmittelgemisches verbleibt ein weisses Kristallpulver; Smp. 234-235°C. Ausbeute 16,6 g (73 mMol; 73 % d.Th.).

### Beispiel 2: Herstellung von 2-Phenylamino-4-chlor-6-cyclopropylpyrimidin der Formel

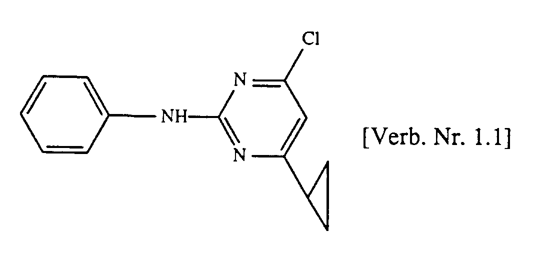

Zu 50 ml (0,55 Mol) Phosphoroxychlorid werden unter Rühren bei 75°C 19,6 g (86,3 mMol) 2-Phenylamino-4-hydroxy-6-cyclopropyl-pyrimidin innerhalb einer halben Stunde zugegeben und anschliessend eine Stunde unter Rückfluss erhitzt. Nach beendeter Chlorwasserstoffentwicklung wird das auf Raumtemperatur abgekühlte Reaktionsgemisch unter kräftigem Rühren in 200 ml Wasser eingetragen, wobei die Temperatur durch Zugabe von Eis auf ca. 50°C gehalten wird. Durch langsames Zutropfen von 220 ml 30%iger wässriger Natronlauge werden die ca. 400 ml der hellbraunen Suspension auf pH 7 gebracht und dreimal mit je 150 ml Ethylacetat extrahiert. Die vereinigten Extrakte werden zweimal mit je 100 ml Wasser gewaschen, über Natriumsulfat getrocknet, filtriert und das Lösungsmittel verdampft. Die 20,7 g hellbraunes Pulver werden mit 400 ml Diethylether behandelt und vom Ungelösten abfiltriert. Nach Abdampfen des Diethylethers wird der Rückstand aus Diisopropylether umkristallisiert. Die gelblichen Kristalle schmelzen bei 127-128°C. Ausbeute 17,7 g (72,1 mMol; 83,5 % d.Th.).

### Beispiel 3: Herstellung von 2-Phenylamino-4-methoxy-6-cyclopropylpyrimidin der Formel

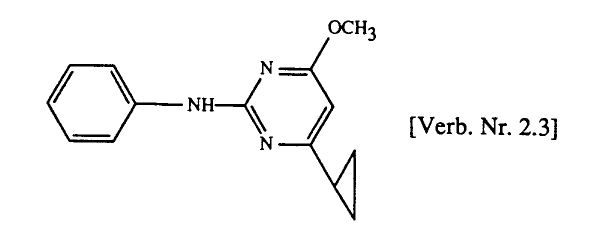

Zu 5,8 g (32,2 mMol) Natriummethylat in 10 ml abs. Methanol werden innerhalb einer halben Stunde unter Rühren bei Raumtemperatur 6,87 g (28 mMol) 2-Phenylamino-4-chlor-6-cyclopropyl-pyrimidin in 25 ml Dioxan zugetropft und anschliessend 6 Stunden auf 50°C erwärmt. Zum Vervollständigen der Reaktion werden noch 0,76 g (4,2 mMol) Natriummethylat zugegeben und weitere 20 Stunden auf 50°C erhitzt. Nach Abdampfen der Lösungsmittel wird der Rückstand mit 300 ml Diethylether versetzt, die Suspension zweimal mit je 100 ml Wasser gewaschen, dann über Natriumsulfat getrocknet, filtriert und der Diethylether abgedampft. 6,7 g zurückbleibendes gelbes Oel werden säulenchromatographisch über Kieselgel mit Toluol als Laufmittel gereinigt. Nach Verdampfen des Toluols verbleibt ein hellgelbes Oel mit dem Brechungsindex nD25: 1,6225. Ausbeute: 5,8 g (24 mMol; 86 % d.Th.).

### Beispiel 4: Herstellung von 2-Phenylamino-4-dimethylamino-6-cyclopropylpyrimidin der Formel

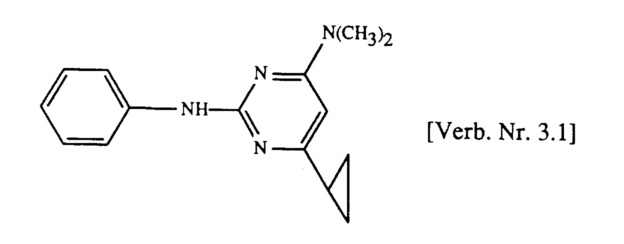

Zu 9,8 g (40 mMol) 2-Phenylamino-4-chlor-6-cyclopropyl-pyrimidin in 20 ml abs. Ethanol werden bei Raumtemperatur unter Rühren innerhalb von 10 Minuten 17,9 ml 33%ige (100 mMol) abs. ethanolische Dimethylaminlösung zugetropft, und die entstandene gelbe Suspension wird eine Stunde bei 50°C, dann eine weitere Stunde unter Rückfluss erhitzt. Nach Abdampfen des Lösungsmittls wird der Rückstand in 200 ml Methylenchlorid aufgenommen, zweimal mit je 100 ml Wasser gewaschen, über Natriumsulfat getrocknet, filtriert und durch Abdampfen von Methylenchlorid befreit. Die zurückbleibenden 10,2 g eines gelben Oels werden säulenchromatographisch über Kieselgel mit Methylenchlorid als Laufmittel gereinigt. Nach Verdampfen des Methylenchlorids bleibt ein hellgelbes Oel zurück, das nach einigen Stunden kristallisiert. Die hellgelben Kristalle schmelzen bei 90-91°C. Ausbeute: 8,9 g (35 mMol; 87,5 % d.Th.).

Auf diese Art oder nach einer der weiter oben angegebenen Methoden werden folgende Verbindungen gewonnen.

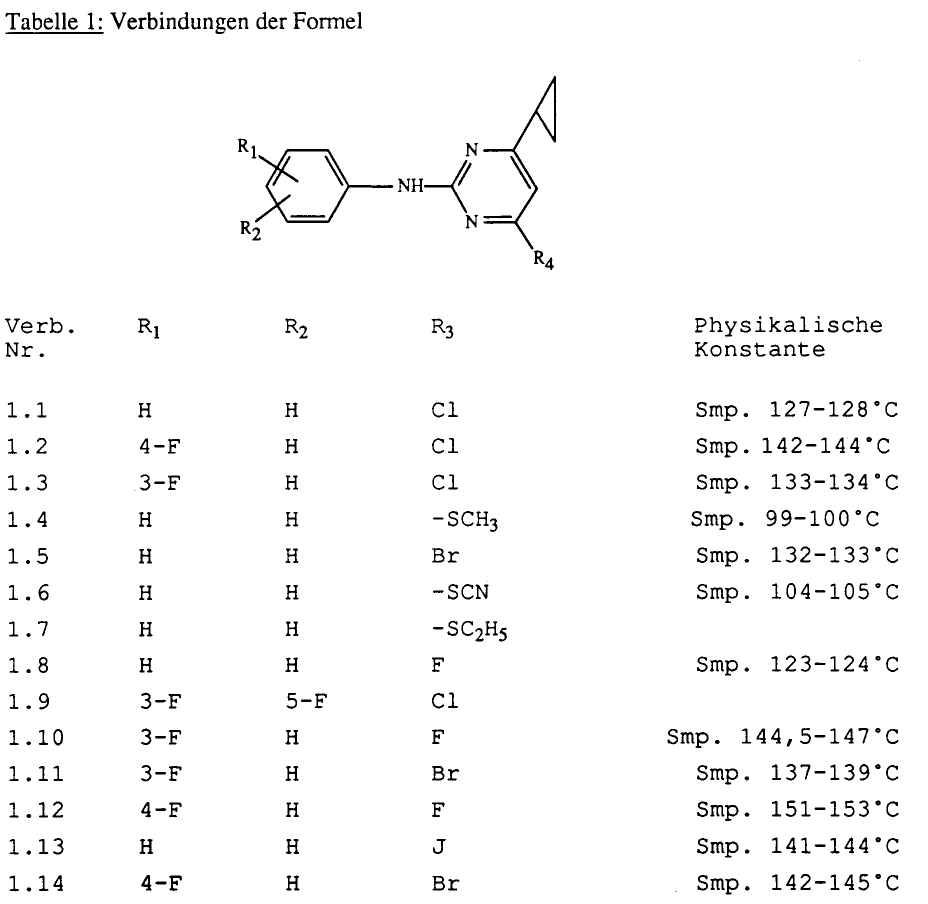

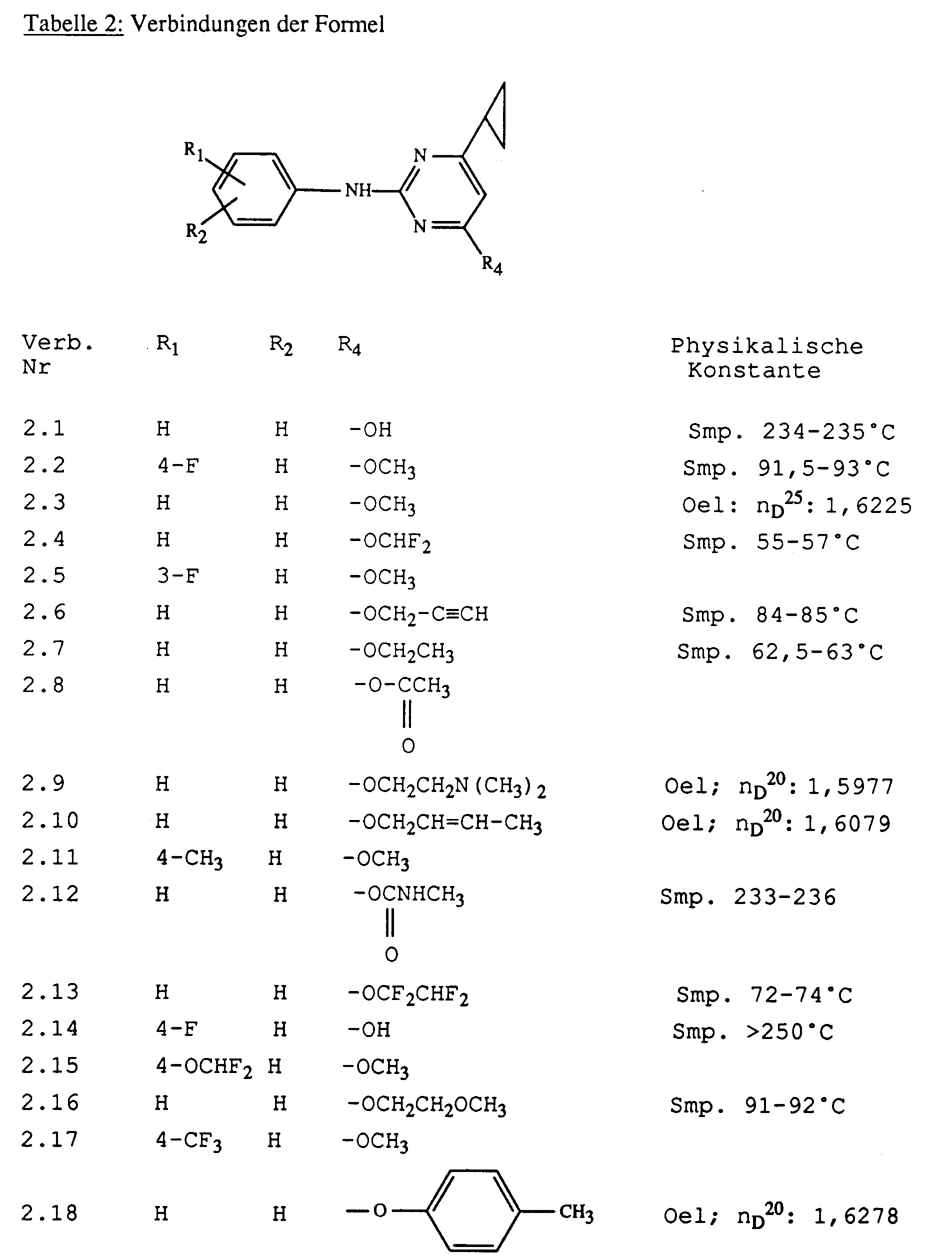

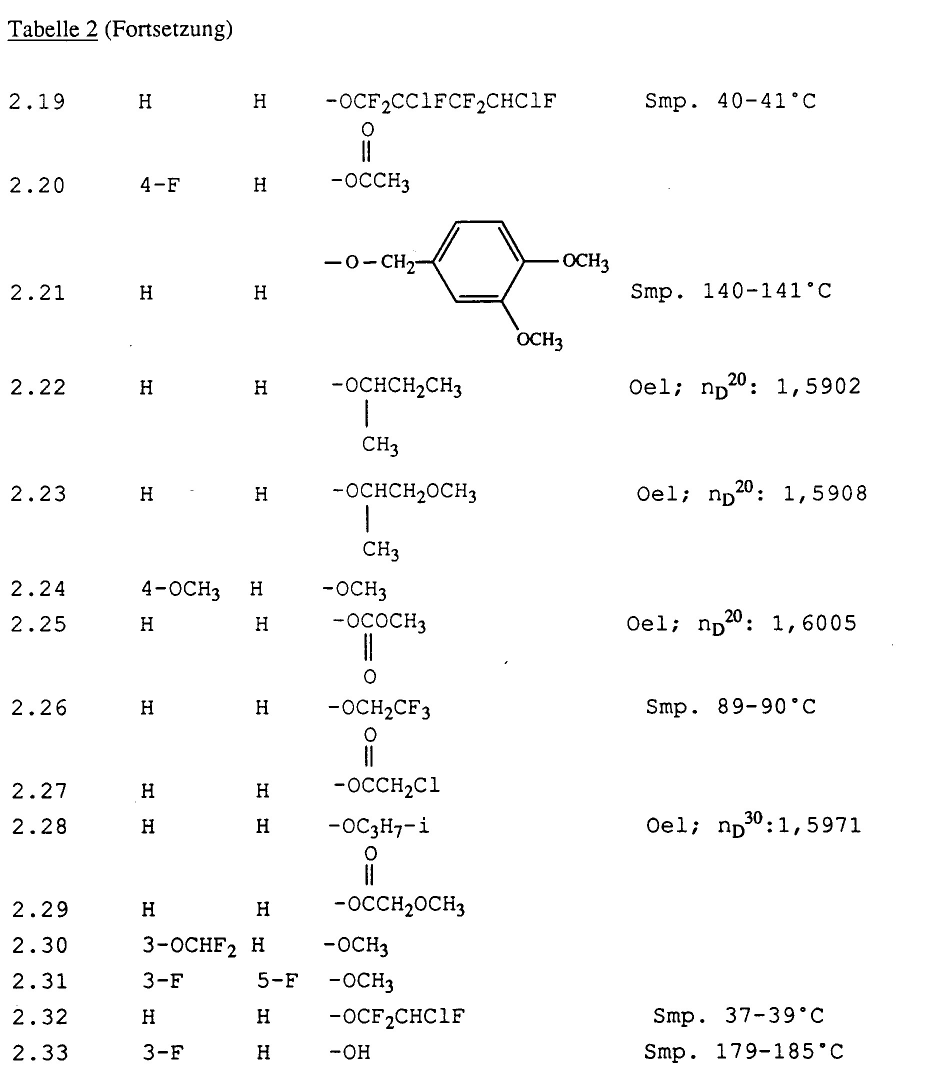

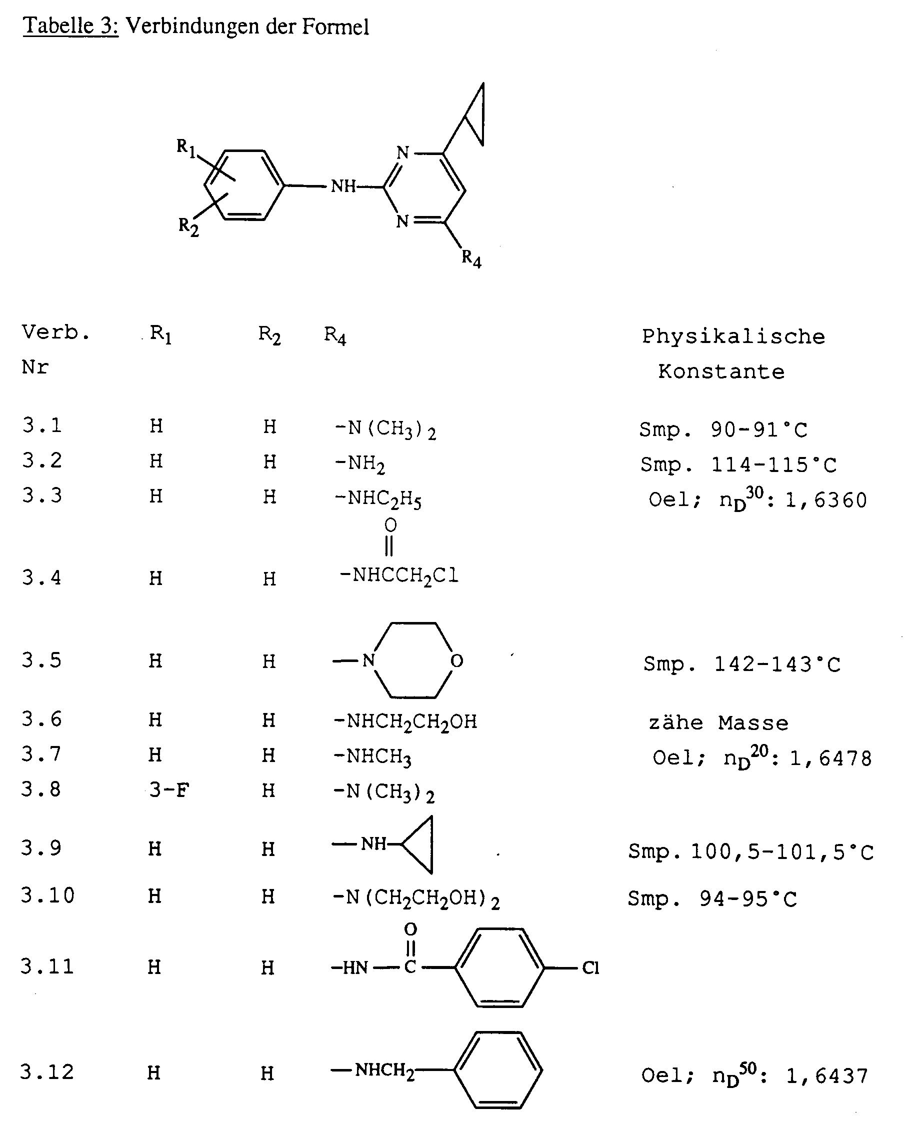

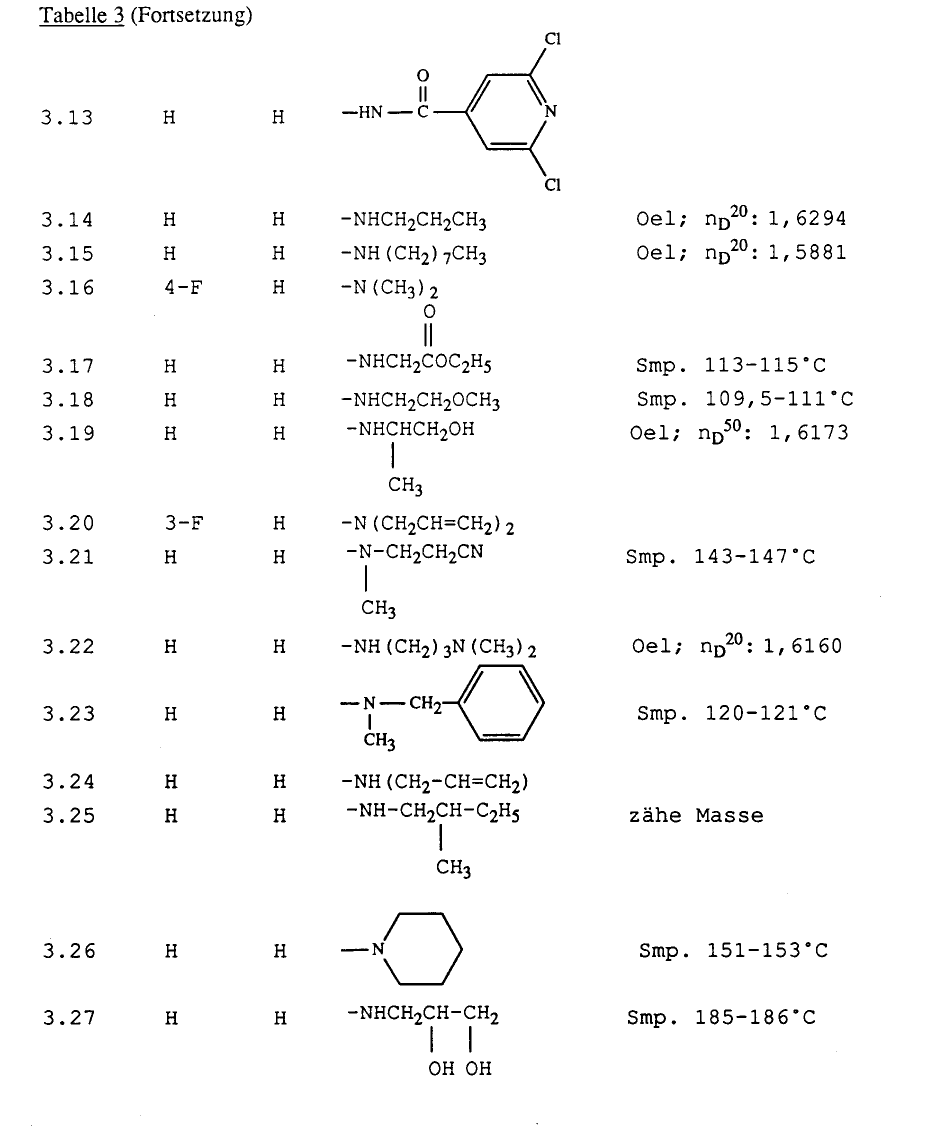

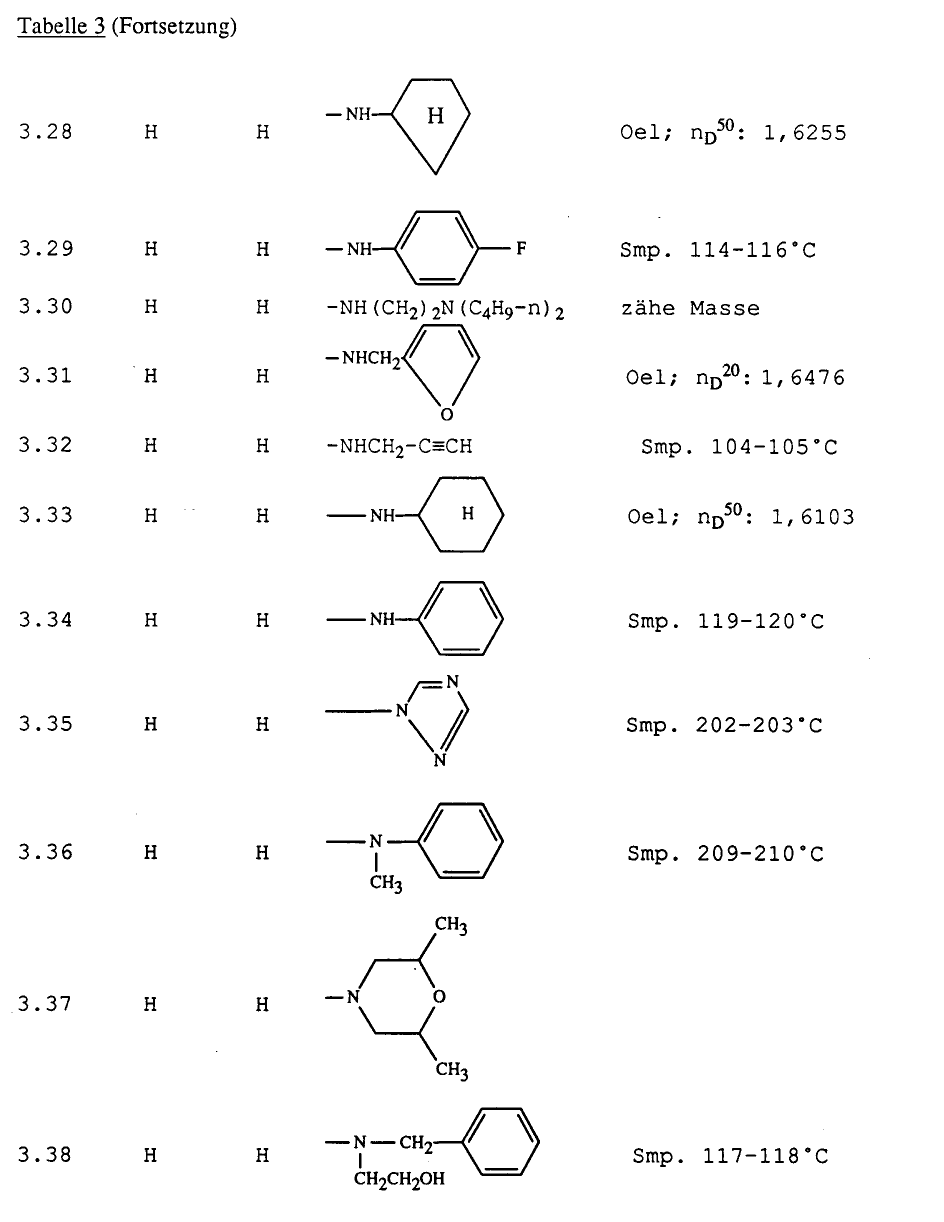

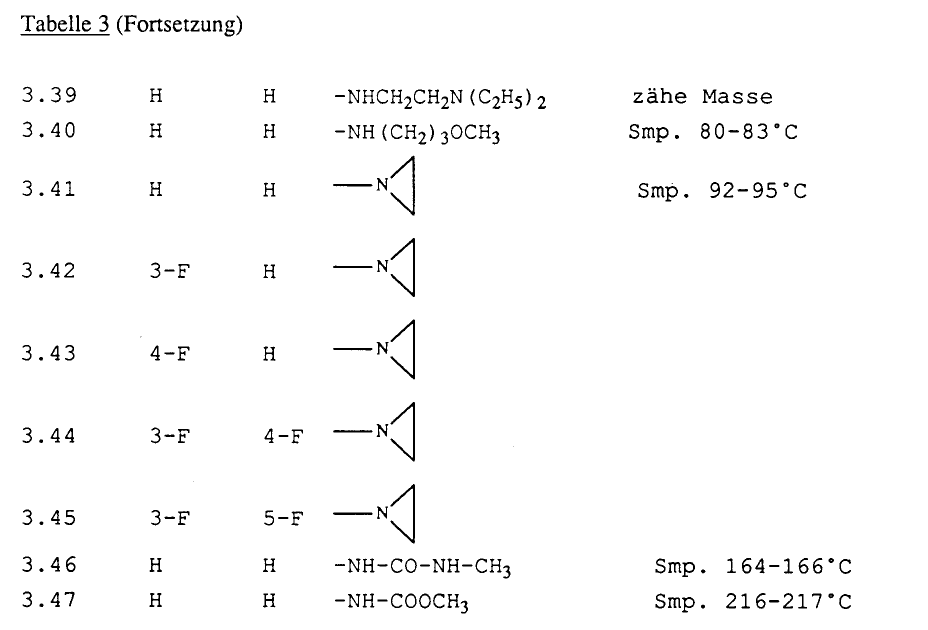

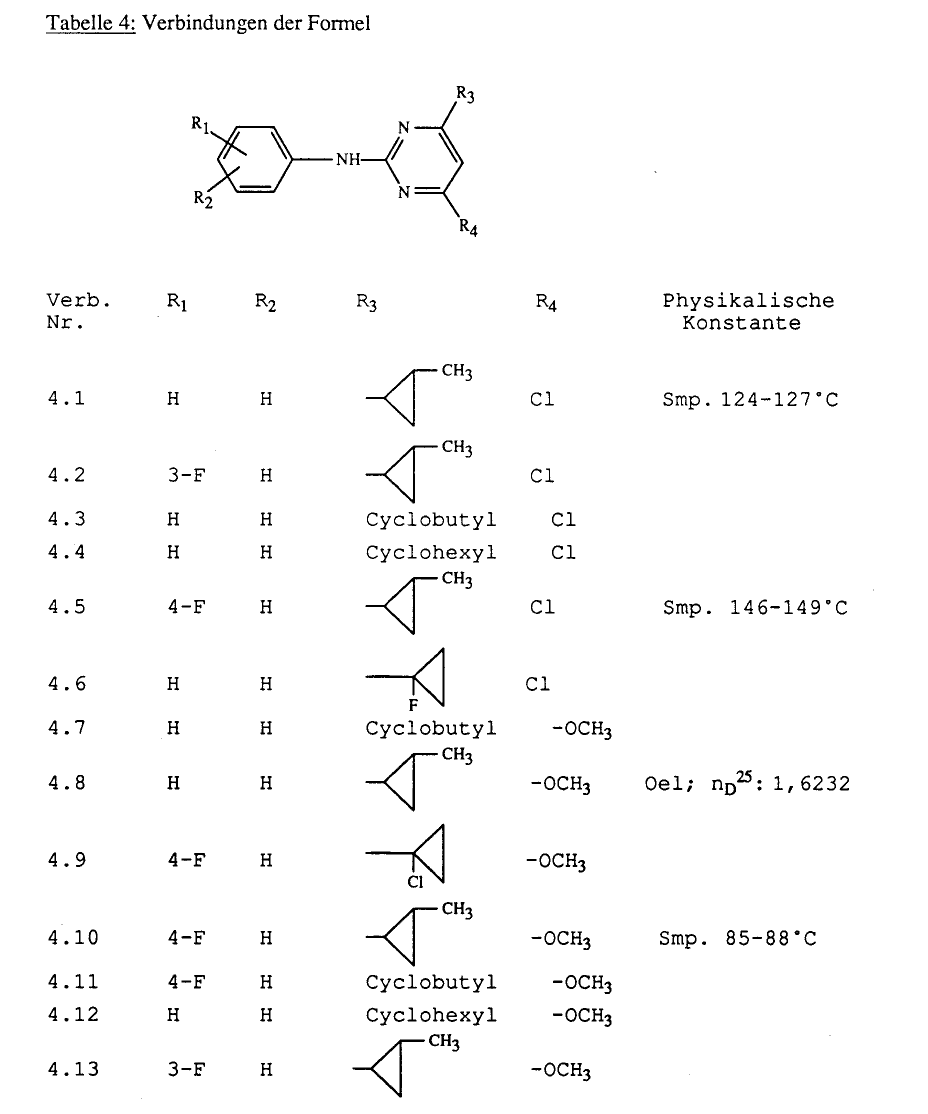

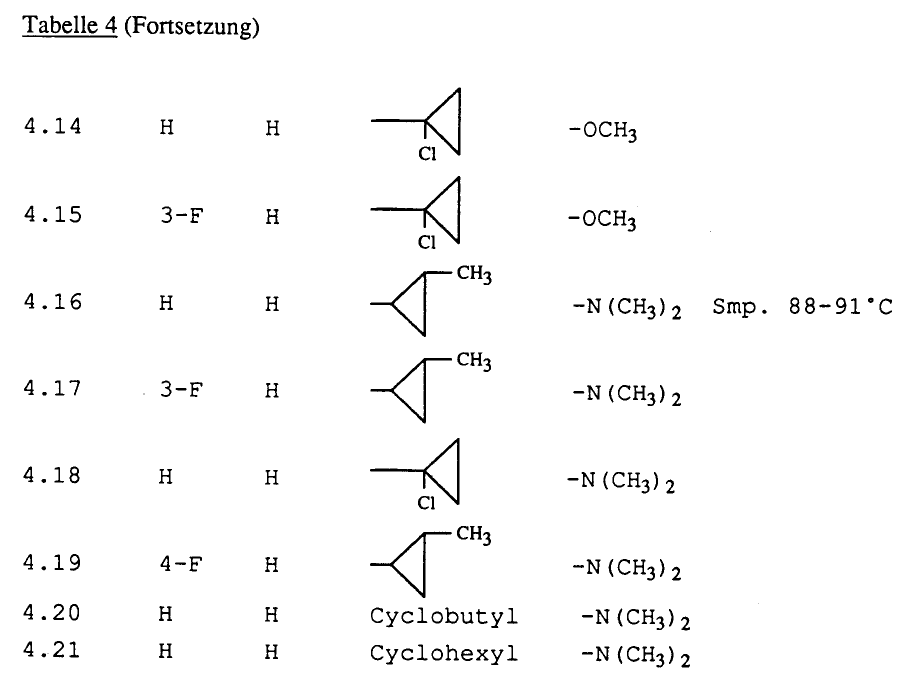

### 2. Formulierungsbeispiele für flüssige Wirkstoffe der Formel I (% = Gewichtsprozent)

| 2.1. Emulsionskonzentrate | a) | b) | c) |
|---|---|---|---|
| Wirkstoff aus der Tabelle | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus der Tabelle | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) (MG = Molekulargewicht) | - | - | 94 % | - |

Die Lösungen sind zur Anwendung in Form kleinster Tropfen geeignet.

| 2.3. Granulate | a) | b) |
|---|---|---|
| Wirkstoff aus der Tabelle | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff aus der Tabelle | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

### Formulierungsbeispiele für feste Wirkstoffe der Formel I(% = Gewichtsprozent)

| 2.5. Spritzpulver | a) | b) | c) |
|---|---|---|---|
| Wirkstoff aus der Tabelle | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6. Emulsions-Konzentrat | |
|---|---|
| Wirkstoff aus der Tabelle | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff aus der Tabelle | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus der Tabelle | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Umhüllungs-Granulat | |
|---|---|
| Wirkstoff aus der Tabelle | 3 % |
| Polyethylenglykol (MG 200) | 3 % |
| Kaolin (MG = Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10. Suspensions-Konzentrat | |
|---|---|
| Wirkstoff aus der Tabelle | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung gegen Venturia inaequalis auf Apfeltrieben. Residual-protektive Wirkung

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Verbindungen aus den Tabellen 1 bis 4 zeigen gegen Venturia gute Wirksamkeit (Befall: weniger als 20 %). So reduzierten z.B. die Verbindungen Nr. 1.1, 1.13 und 2.2 den Venturia-Befall auf 0 bis 5 %. Unbehandelte aber infizierte Kontrollpflanzen wiesen dagegen einen Venturia-Befall von 100 % auf.

### Beispiel 3.2: Wirkung gegen Botrytis cinerea an Apfelfrüchten. Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Verbindungen aus den Tabellen zeigen gegen Botrytis gute Wirksamkeit (Befall: weniger als 20 %). So reduzierten z.B. die Verbindungen Nr. 1.1, 1.2, 1.3, 1.5, 1.10, 1.11, 1.12, 1.13, 1.14, 2.2, 2.3, 2.4, 2.13, 2.28, 3.1, 3.9, 3.21, 3.41, 4.1, 4.5, 4.8, 4.10, 4.16 und andere den Botrytis-Befall auf 0 bis 10%. Unbehandelte aber infizierte Kontrollpflanzen wiesen dagegen einen Botrytis-Befall von 100 % auf.

### Beispiel 3.3: Wirkung gegen Erysiphae graminis auf Gerste

### a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus den Tabellen zeigen gegen Erysiphae gute Wirksamkeit (Befall: weniger als 20 %). Unbehandelte aber infizierte Kontrollpflanzen weisen dagegen einen Erysiphae-Befall von 100 % auf.

### Beispiel 3.4: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabellen 1 bis 4 verhindern den Pilzbefall weitgehend (0 bis 10 % Pilzbefall).

### Beispiel 3.5: Wirkung gegen Colletotrichum lagenarium auf Gurken

Gurkenpflanzen werden nach 2-wöchiger Anzucht mit einer aus Spritzpulver des Wirkstoffs hergestellten Spritzbrühe (Konzentration 0,002 %) besprüht. Nach 2 Tagen werden die Pflanzen mit einer Sporensuspension (1,5x10⁵ Sporen/ml) des Pilzes infiziert und für 36 Stunden bei 23°C und hoher Luftfeuchtigkeit inkubiert. Die Inkubation wird dann bei normaler Luftfeuchtigkeit und ca. 22-23°C weitergeführt. Der eingetretene Pilzbefall wird 8 Tage nach der Infektion beurteilt. Unbehandelte aber infizierte Kontrollpflanzen weisen einen Pilzbefall von 100 % auf.

Verbindungen der Formel I aus den Tabellen zeigen gute Wirksamkeit und verhindern die Ausbreitung des Krankheitsbefalls. Der Pilzbefall wird auf 20 % oder weniger zurückgedrängt.

## Patentansprüche (Patentansprüche für folgende(n) Vertragsstaat(en): CH, DE, DK, FR, GB, IT, LI, NL)

1. Verbindungen der Formel I 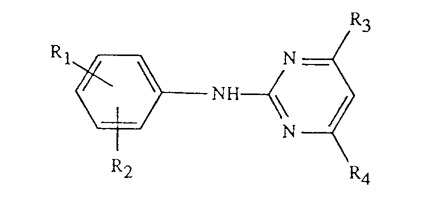 in welcher bedeuten:
R₁ und R₂ Wasserstoff;
R₃ C₃-C₆-Cycloalkyl oder durch Methyl oder Halogen bis zu dreifach gleich oder verschieden substituiertes C₃-C₆-Cycloalkyl;
R₄ Halogen, Thiocyano(-SCN), -OR₅, -SR₅, -NR₅R₆, wobei
R₅
a) - Wasserstoff, unsubstituiertes C₁-C₈-Alkyl, oder aber eine C₁-C₄-Alkylgruppe darstellt, die durch Halogen, Hydroxy, Cyano, C₁-C₄-Alkoxy, C₁-C₄-Alkylamino, Bis(C₁-C₄-Alkyl)amino, C₃-C₆-Cycloalkyl, oder durch substituiertes oder unsubstituiertes Phenyl oder durch -CO-OC₁-C₃-Alkyl substituiert ist; oder
b) - unsubstituiertes oder durch Methyl substituiertes C₃-C₆-Cycloalkyl darstellt; oder
c) - unsubstituiertes oder durch Halogen substituiertes C₃-C₆-Alkenyl darstellt; oder
d) - unsubstituiertes oder durch Halogen substituiertes C₃-C₆-Alkinyl darstellt; oder
e) - unsubstituiertes oder durch Halogen, C₁-C₃-Alkyl, C₁-C₃-Alkoxy, Nitro substituiertes Phenyl darstellt; oder
f) - einen unsubstituierten oder durch Halogen, C₁-C₃-Alkyl, C₁-C₃-Alkoxy, Nitro substituierten 5-gliedrigen oder 6-gliedrigen gegebenenfalls über -CH₂-gebundenen heterocyclischen Rest mit ein bis drei Heteroatomen N, O oder S darstellt; oder
g) - einen Acylrest -CO-R' darstellt, wobei R' unsubstituiertes oder durch Halogen, C₁-C₃-Alkoxy substituiertes C₁-C₆-Alkyl; unsubstituiertes oder durch Halogen substituiertes C₃-C₆-Alkenyl; unsubstituiertes oder durch Halogen, C₁-C₃-Alkyl, C₁-C₃-Alkoxy, Nitro substituiertes Phenyl bedeutet; oder
h) - einen Carbamoylrest -CO-NH-R'' oder einen Oxycarbonylrest -CO-OR'' darstellt, in welchem R'' einen aliphatischen oder cycloaliphatischen Rest mit maximal 6 C-Atomen bedeutet, der unsubstituiert oder halogensubstituiert ist, oder worin R'' einen Phenyl- oder Benzylrest bedeutet, der je unsubstituiert oder im aromatischen Ring durch Halogen, C₁-C₃-Alkyl, C₁-C₃-Alkoxy, Nitro substituiert ist; und worin
R₆ Wasserstoff, unsubstituiertes C₁-C₈-Alkyl oder aber C₁-C₄-Alkyl darstellt, das durch Halogen, Hydroxy, Cyano, C₁-C₄-Alkoxy substituiert ist; oder worin R₆ unsubstituiertes oder durch Methyl substituiertes C₃-C₆-Cycloalkyl; je unsubstituiertes oder durch Halogen substituiertes C₃-C₆-Alkenyl oder C₃-C₆-Alkinyl; oder unsubstituiertes oder durch Halogen, C₁-C₃-Alkyl, C₁-C₃-Alkoxy, Nitro substituiertes Phenyl darstellt; wobei im Falle R₄ = NR₅R₆ die Substituenten R₅ und R₆ zusammen mit dem N-Atom auch gemeinsam einen Aziridinring oder einen 5- oder 6-gliedrigen heterocyclischen Rest bilden können, der zusätzlich zu dem N-Atom noch einen oder zwei Heteroatome N, O oder S enthalten und alkylsubstituiert sein kann.

2. Verbindungen gemäss Anspruch 1, worin R₁ und R₂ Wasserstoff bedeutet; und
R₃ C₃-C₄-Cycloalkyl, das unsubstituiert oder durch Methyl oder Halogen monosubstituiert ist;
R₄ Halogen, -OR₅, -NR₅R₆ bedeuten, wobei
R₅ die Bedeutungen a), b), c), d) oder g) hat, und
R₆ Wasserstoff oder einen Alkyl-, Alkenyl- oder Alkinylrest darstellt, der wahlweise unsubstituiert oder halogensubstituiert ist; oder wobei
R₅ und R₆ zusammen mit dem gemeinsamen N-Atom einen Aziridinring, oder einen wahlweise unsubstituierten oder methylsubstituierten Piperidin- oder Morpholinring darstellen.

3. Verbindungen gemäss Anspruch 1, worin bedeuten:
R₁ und R₂ Wasserstoff;
R₃ Cyclopropyl oder durch Methyl oder Halogen ein- oder zweifach gleich oder verschieden substituiertes Cyclopropyl;
R₄ Halogen, -SR₅, -OR₅, -NR₅R₆; und
R₅ Wasserstoff oder eine C₁-C₄-Alkylgruppe darstellt, die unsubstituiert oder durch Halogen, Hydroxy, C₁-C₄-Alkoxy, C₁-C₄-Alkylamino, Bis(C₁-C₄-Alkyl)amino, Cyclopropyl oder durch -COOC₁-C₃-Alkyl substituiert ist; oder worin R₅ C₃-C₄-Alkenyl oder C₃-C₄-Alkinyl darstellt oder eine unsubstituierte oder durch Halogen, Methyl, oder Methoxy substituierte Phenylgruppe oder einen Acylrest gemäss der Bedeutung g) darstellt; und R₆ Wasserstoff oder eine C₁-C₄-Alkylgruppe darstellt, die unsubstituiert oder durch Halogen, Hydroxy, C₁-C₄-Alkoxy, C₁-C₄-Alkylamino oder Bis(C₁-C₄-Alkyl)amino substituiert ist oder worin R₆ C₃-C₄-Alkenyl oder C₃-C₄-Alkinyl darstellt.

4. Verbindungen gemäss Anspruch 3, bei denen
R₁ und R₂ Wasserstoff bedeutet,
R₃ Cyclopropyl darstellt,
R₄ Halogen, -OR₅ oder -NR₅R₆ ist, und
R₅ Wasserstoff oder R₆ bedeutet, und
R₆ C₁-C₃-Alkyl darstellt, das unsubstituiert oder durch C₁-C₄-Alkoxy oder Cyclopropyl monosubstituiert oder durch Halogen bis zu fünffach substituiert ist; oder C₃-C₄-Alkenyl oder Alkinyl bedeutet.

5. Verbindungen gemäss Anspruch 1, worin R₁ und R₂ Wasserstoff bedeutet; und
R₃ C₃-C₄-Cycloalkyl, das unsubstituiert oder durch Methyl oder Halogen monosubstituiert ist und
R₄ Halogen bedeuten.

6. Verfahren zur Herstellung von Verbindungen der Formel I, dadurch gekennzeichnet, dass man
a) ein Phenylguanidinsalz der Formel IIa 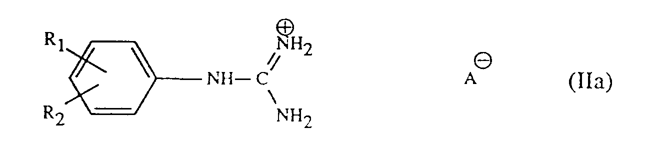 worin A^{⊖} ein beliebiges Anion bedeutet oder das zugrundeliegende Guanidin der Formel IIb 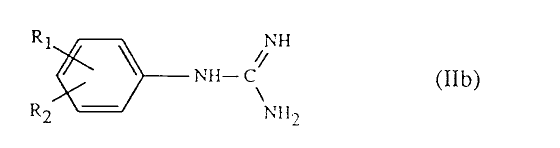 mit einem β-Ketoester der Formel III, 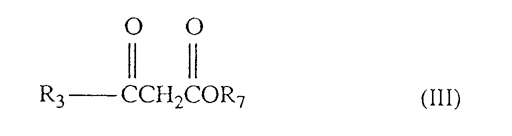 worin R₁, R₂, R₃ die für Formel I gegebene Bedeutung haben, und R₇ C₃-C₆-Alkenyl, C₃-C₆-Alkinyl, unsubstituiertes oder mit Halogen und/oder C₁-C₃-Alkyl substituiertes Benzyl oder C₁-C₈-Alkyl bedeuten, ohne Lösungsmittel oder in einem Lösungsmittel, bei Temperaturen von 60°C bis 160°C, zu der Verbindung Ia umsetzt; und, sofern gewünscht die OH-Gruppe acyliert, carbamoyliert oder carbonyliert, oder gegen Halogen zur Gewinnung von Verbindungen der Formel Ib austauscht, und, sofern gewünscht, weiteren Austausch des Halogenatoms gegen eine der übrigen für R₄ genannten Gruppen 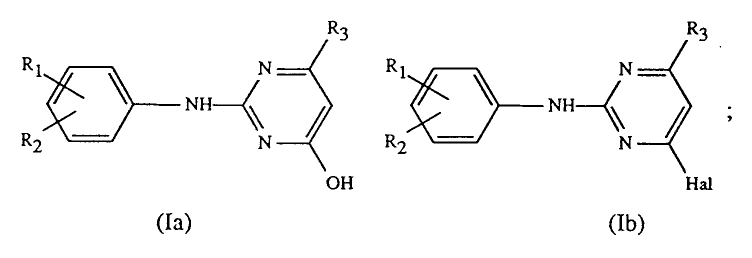 oder
b) dass man ein Isothiuroniumsalz der Formel IV 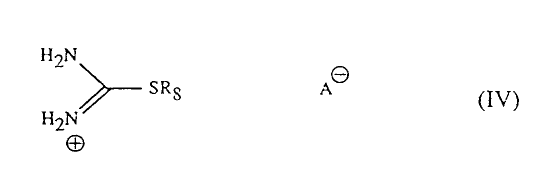 wobei A^{⊖} ein beliebiges Anion und R₈ C₁-C₈-Alkyl oder unsubstituiertes oder mit Halogen und/oder C₁-C₄-Alkyl substituiertes Benzyl bedeuten, mit einem β-Ketoester der Formel III, bevorzugt in einem Lösungsmittel, bei Temperaturen von 40°C bis 140°C zur Reaktion bringt, wobei sich das Thiopyrimidin V bildet, 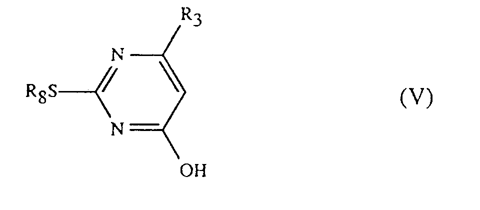 das mit einem Oxydationsmittel zu der Pyrimidinverbindung der Formel VI 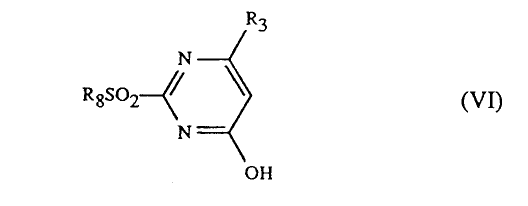 oxydiert wird und dieses mit einem Formylanilin der Formel VII 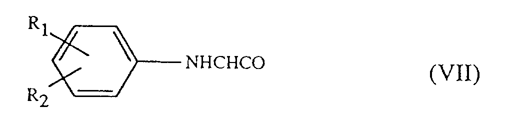 in einem inerten Lösungsmittel in Gegenwart einer Base bei Temperaturen zwischen -30°C bis +120°C zu einer Verbindung der Formel VIII 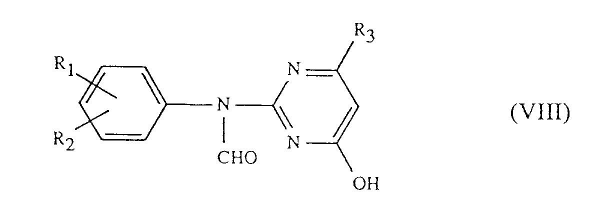 umgesetzt wird, woraufhin die erhaltene Verbindung VIII einer Hydrolyse in Gegenwart einer Base oder Säure bei Temperaturen von 10°C bis 110°C unterworfen wird, unter Gewinnung von Verbindungen der Formel Ia, und, sofern gewünscht, Austausch der OH-Gruppe gegen Halogen zur Gewinnung von Verbindungen der Formel Ib, und sofern gewünscht, weiteren Austausch des Halogens gegen eine der übrigen für R₄ genannten Gruppen.

7. Mikrobizides Mittel enthaltend neben üblichem Trägermaterial als aktive Komponente eine Verbindung der Formel I gemäss einem der Ansprüche 1-5.

8. Verfahren zur Bekämpfung oder Verhütung eines Befalls von Kulturpflanzen durch phytopathogene Mikroorganismen, dadurch gekennzeichnet, dass man als Wirkstoff eine Verbindung der Formel I gemäss einem der Ansprüche 1-5 auf die Pflanze, auf Pflanzenteile oder ihren Standort appliziert.

## Patentansprüche (Patentansprüche für folgende(n) Vertragsstaat(en): ES)

1. Verfahren zur Herstellung von Verbindungen der Formel I 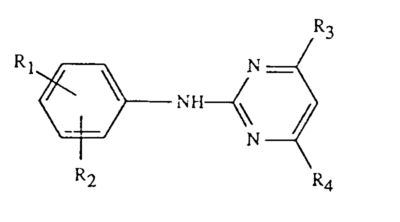 in welcher bedeuten:
R₁ und R₂ Wasserstoff;
R₃ C₃-C₆-Cycloalkyl oder durch Methyl oder Halogen bis zu dreifach gleich oder verschieden substituiertes C₃-C₆-Cycloalkyl;
R₄ Halogen, Thiocyano(-SCN), -OR₅, -SR₅, -NR₅R₆, wobei
R₅
a) - Wasserstoff, unsubstituiertes C₁-C₈-Alkyl, oder aber eine C₁-C₄-Alkylgruppe darstellt, die durch Halogen, Hydroxy, Cyano, C₁-C₄-Alkoxy, C₁-C₄-Alkylamino, Bis(C₁-C₄-Alkyl)amino, C₃-C₆-Cycloalkyl, oder durch substituiertes oder unsubstituiertes Phenyl oder durch -CO-OC₁-C₃-Alkyl substituiert ist; oder
b) - unsubstituiertes oder durch Methyl substituiertes C₃-C₆-Cycloalkyl darstellt; oder
c) - unsubstituiertes oder durch Halogen substituiertes C₃-C₆-Alkenyl darstellt; oder
d) - unsubstituiertes oder durch Halogen substituiertes C₃-C₆-Alkinyl darstellt; oder
e) - unsubstituiertes oder durch Halogen, C₁-C₃-Alkyl, C₁-C₃-Alkoxy, Nitro substituiertes Phenyl darstellt; oder
f) - einen unsubstituierten oder durch Halogen, C₁-C₃-Alkyl, C₁-C₃-Alkoxy, Nitro substituierten 5-gliedrigen oder 6-gliedrigen gegebenenfalls über -CH₂-gebundenen heterocyclischen Rest mit ein bis drei Heteroatomen N, O oder S darstellt; oder
g) - einen Acylrest -CO-R' darstellt, wobei R' unsubstituiertes oder durch Halogen, C₁-C₃-Alkoxy substituiertes C₁-C₆-Alkyl; unsubstituiertes oder durch Halogen substituiertes C₃-C₆-Alkenyl; unsubstituiertes oder durch Halogen, C₁-C₃-Alkyl, C₁-C₃-Alkoxy, Nitro substituiertes Phenyl bedeutet; oder
h) - einen Carbamoylrest -CO-NH-R'' oder einen Oxycarbonylrest -CO-OR'' darstellt, in welchem R'' einen aliphatischen oder cycloaliphatischen Rest mit maximal 6 C-Atomen bedeutet, der unsubstituiert oder halogensubstituiert ist, oder worin R'' einen Phenyl- oder Benzylrest bedeutet, der je unsubstituiert oder im aromatischen Ring durch Halogen, C₁-C₃-Alkyl, C₁-C₃-Alkoxy, Nitro substituiert ist; und worin
R₆ Wasserstoff, unsubstituiertes C₁-C₈-Alkyl oder aber C₁-C₄-Alkyl darstellt, das durch Halogen, Hydroxy, Cyano, C₁-C₄-Alkoxy substituiert ist; oder worin R₆ unsubstituiertes oder durch Methyl substituiertes C₃-C₆-Cycloalkyl; je unsubstituiertes oder durch Halogen substituiertes C₃-C₆-Alkenyl oder C₃-C₆-Alkinyl; oder unsubstituiertes oder durch Halogen, C₁-C₃-Alkyl, C₁-C₃-Alkoxy, Nitro substituiertes Phenyl darstellt; wobei im Falle R₄ = NR₅R₆ die Substituenten R₅ und R₆ zusammen mit dem N-Atom auch gemeinsam einen Aziridinring oder einen 5- oder 6-gliedrigen heterocyclischen Rest bilden können, der zusätzlich zu dem N-Atom noch einen oder zwei Heteroatome N, O oder S enthalten und alkylsubstituiert sein kann, dadurch gekennzeichnet dass man
a) ein Phenylguanidinsalz der Formel IIa 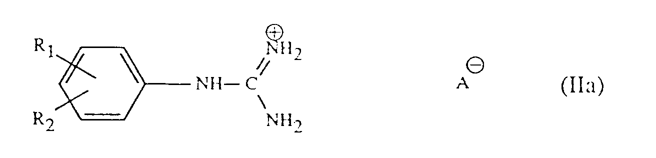 worin A^{⊖} ein beliebiges Anion bedeutet oder das zugrundeliegende Guanidin der Formel IIb 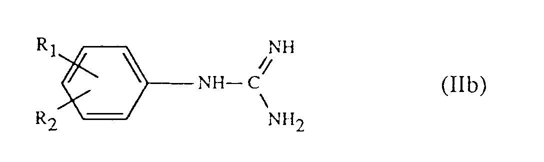 mit einem β-Ketoester der Formel III, 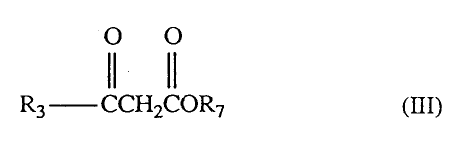 worin R₁, R₂, R₃ die für Formel I gegebene Bedeutung haben, und R₇ C₃-C₆-Alkenyl, C₃-C₆-Alkinyl, unsubstituiertes oder mit Halogen und/oder C₁-C₃-Alkyl substituiertes Benzyl oder C₁-C₈-Alkyl bedeuten, ohne Lösungsmittel oder in einem Lösungsmittel, bei Temperaturen von 60°C bis 160°C, zu der Verbindung Ia umsetzt; und, sofern gewünscht die OH-Gruppe acyliert, carbamoyliert oder carbonyliert, oder gegen Halogen zur Gewinnung von Verbindungen der Formel Ib austauscht, und, sofern gewünscht, weiteren Austausch des Halogenatoms gegen eine der übrigen für R₄ genannten Gruppen 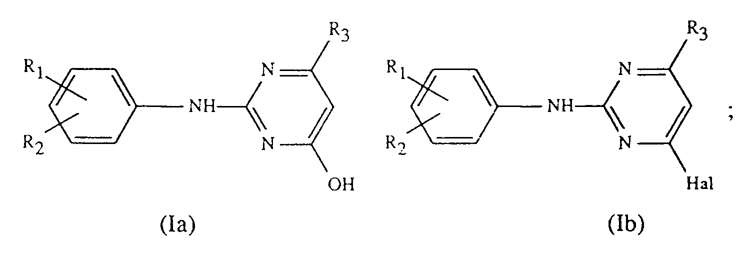 oder
b) dass man ein Isothiuroniumsalz der Formel IV 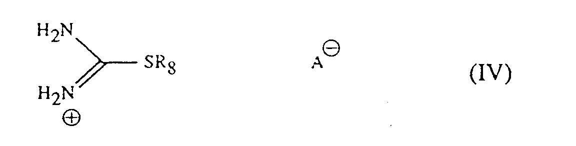 worin A^{⊖} ein beliebiges Anion und R₈ C₁-C₈-Alkyl oder unsubstituiertes oder mit Halogen und/oder C₁-C₄-Alkyl substituiertes Benzyl bedeuten, mit einem β-Ketoester der Formel III, bevorzugt in einem Lösungsmittel, bei Temperaturen von 40°C bis 140°C zur Reaktion bringt, wobei sich das Thiopyrimidin V bildet, 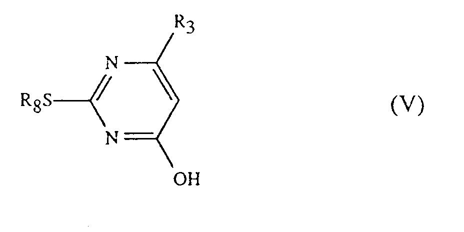 das mit einem Oxydationsmittel zu der Pyrimidinverbindung der Formel VI 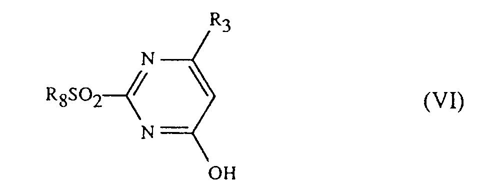 oxydiert wird und dieses mit einem Formylanilin der Formel VII 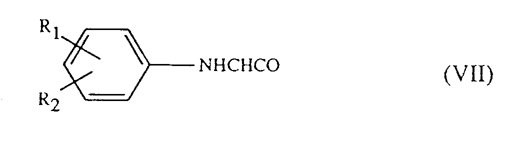 in einem inerten Lösungsmittel in Gegenwart einer Base bei Temperaturen zwischen -30°C bis +120°C zu einer Verbindung der Formel VIII 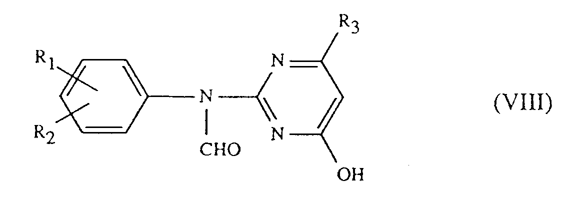 umgesetzt wird, woraufhin die erhaltene Verbindung VIII einer Hydrolyse in Gegenwart einer Base oder Säure bei Temperaturen von 10°C bis 110°C unterworfen wird, unter Gewinnung von Verbindungen der Formel Ia, und, sofern gewünscht, Austausch der OH-Gruppe gegen Halogen zur Gewinnung von Verbindungen der Formel Ib, und sofern gewünscht, weiteren Austausch des Halogens gegen eine der übrigen für R₄ genannten Gruppen.

2. Verfahren gemäss Anspruch 1, worin R₁ und R₂ Wasserstoff bedeutet; und
R₃ C₃-C₄-Cycloalkyl, das unsubstituiert oder durch Methyl oder Halogen monosubstituiert ist;
R₄ Halogen, -OR₅, -NR₅R₆ bedeuten, wobei
R₅ die Bedeutungen a), b), c), d) oder g) hat, und
R₆ Wasserstoff oder einen Alkyl-, Alkenyl- oder Alkinylrest darstellt, der wahlweise unsubstituiert oder halogensubstituiert ist; oder wobei
R₅ und R₆ zusammen mit dem gemeinsamen N-Atom einen Aziridinring, oder einen wahlweise unsubstituierten oder methylsubstituierten Piperidin- oder Morpholinring darstellen.

3. Verfahren gemäss Anspruch 1, worin bedeuten:
R₁ und R₂ Wasserstoff;
R₃ Cyclopropyl oder durch Methyl oder Halogen ein- oder zweifach gleich oder verschieden substituiertes Cyclopropyl;
R₄ Halogen, -SR₅, -OR₅, -NR₅R₆; und
R₅ Wasserstoff oder eine C₁-C₄-Alkylgruppe darstellt, die unsubstituiert oder durch Halogen, Hydroxy, C₁-C₄-Alkoxy, C₁-C₄-Alkylamino, Bis(C₁-C₄-Alkyl)amino, Cyclopropyl oder durch -COOC₁-C₃-Alkyl substituiert ist; oder worin R₅ C₃-C₄-Alkenyl oder C₃-C₄-Alkinyl darstellt oder eine unsubstituierte oder durch Halogen, Methyl, oder Methoxy substituierte Phenylgruppe oder einen Acylrest gemäss der Bedeutung g) darstellt; und R₆ Wasserstoff oder eine C₁-C₄-Alkylgruppe darstellt, die unsubstituiert oder durch Halogen, Hydroxy, C₁-C₄-Alkoxy, C₁-C₄-Alkylamino oder Bis(C₁-C₄-Alkyl)amino substituiert ist oder worin R₆ C₃-C₄-Alkenyl oder C₃-C₄-Alkinyl darstellt.

4. Verfahren gemäss Anspruch 3, bei denen
R₁ und R₂ Wasserstoff bedeutet,
R₃ Cyclopropyl darstellt,
R₄ Halogen, -OR₅ oder -NR₅R₆ ist, und
R₅ Wasserstoff oder R₆ bedeutet, und
R₆ C₁-C₃-Alkyl darstellt, das unsubstituiert oder durch C₁-C₄-Alkoxy oder Cyclopropyl monosubstituiert oder durch Halogen bis zu fünffach substituiert ist; oder C₃-C₄-Alkenyl oder Alkinyl bedeutet.

5. Verfahren gemäss Anspruch 1, worin R₁ und R₂ Wasserstoff, bedeutet; und
R₃ C₃-C₄-Cycloalkyl, das unsubstituiert oder durch Methyl oder Halogen monosubstituiert ist und
R₄ Halogen bedeuten.

## Claims (Claims for the following Contracting State(s): CH, DE, DK, FR, GB, IT, LI, NL)

1. A compound of the formula I 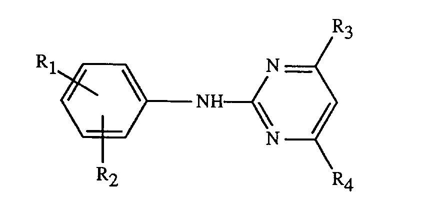 in which: R₁ and R₂ are hydrogen; R₃ is C₃-C₆cycloalkyl or C₃-C₆cycloalkyl which is substituted by up to three identical or different methyl or halogen groups; R₄ is halogen, thiocyano(-SCN), -OR₅, -SR₅ or -NR₅R₆, in which R₅
a) - is hydrogen, unsubstituted C₁-C₈alkyl or a C₁-C₄alkyl group which is substituted by halogen, hydroxyl, cyano, C₁-C₄alkoxy, C₁-C₄alkylamino, bis(C₁-C₄alkyl)amino or C₃-C₆cycloalkyl or by substituted or unsubstituted phenyl or by -CO-OC₁-C₃alkyl; or
b) - is C₃-C₆cycloalkyl which is unsubstituted or substituted by methyl; or
c) - is C₃-C₆alkenyl which is unsubstituted or substituted by halogen; or
d) - is C₃-C₆alkynyl which is unsubstituted or substituted by halogen; or
e) - is phenyl which is unsubstituted or substituted by halogen, C₁-C₃alkyl, C₁-C₃alkoxy or nitro; or
f) - is a 5-membered or 6-membered heterocyclic radical which is bonded via -CH₂- if appropriate, contains one to three hetero atoms N, O or S and is unsubstituted or substituted by halogen, C₁-C₃alkyl, C₁-C₃alkoxy or nitro; or
g) - is an acyl radical -CO-R', in which R' is C₁-C₆alkyl which is unsubstituted or substituted by halogen or C₁-C₃alkoxy; C₃-C₆alkenyl which is unsubstituted or substituted by halogen; or phenyl which is unsubstituted or substituted by halogen, C₁-C₃alkyl, C₁-C₃alkoxy or nitro; or
h) - is a carbamoyl radical -CO-NH-R'' or an oxycarbonyl radical -CO-OR'', in which R'' is an aliphatic or cycloaliphatic radical having not more than 6 C atoms, which is unsubstituted or halogen-substituted, or in which R'' is a phenyl or benzyl radical, which is in each case unsubstituted or substituted in the aromatic ring by halogen, C₁-C₃alkyl, C₁-C₃alkoxy or nitro; and in which
R₆ is hydrogen, unsubstituted C₁-C₈alkyl or C₁-C₄alkyl which is substituted by halogen, hydroxyl, cyano or C₁-C₄alkoxy; or in which R₆ is C₃-C₆cycloalkyl which is unsubstituted or substituted by methyl; C₃-C₆alkenyl or C₃-C₆alkynyl which is in each case unsubstituted or substituted by halogen; or phenyl which is unsubstituted or substituted by halogen, C₁-C₃alkyl, C₁-C₃alkoxy or nitro; and wherein, in the case where R₄ = NR₅R₆, the substituents R₅ and R₆, together with the N atom, can also together form an aziridine ring or a 5- or 6-membered heterocyclic radical, which can also contain one or two hetero atoms N, O or S in addition to the N atom and can be alkyl-substituted.

2. A compound according to claim 1, in which R₁ and R₂ are hydrogen; and R₃ is C₃-C₄cycloalkyl, which is unsubstituted or monosubstituted by methyl or halogen; and R₄ is halogen, -OR₅ or -NR₅R₆, in which R₅ has the meanings a), b), c), d) or g) and R₆ is hydrogen or an alkyl, alkenyl or alkynyl radical, which can be unsubstituted or halogen-substituted if appropriate; or in which R₅ and R₆, together with the common N atom, are an aziridine ring or a piperidine or morpholine ring which is unsubstituted or methyl-substituted if appropriate.

3. A compound according to claim 1, in which: R₁ and R₂ are hydrogen; R₃ is cyclopropyl or cyclopropyl which is substituted by one or two identical or different methyl or halogen groups; R₄ is halogen, -SR₅, -OR₅ or -NR₅R₆; and R₅ is hydrogen or a C₁-C₄alkyl group, which is unsubstituted or substituted by halogen, hydroxyl, C₁-C₄alkoxy, C₁-C₄alkylamino, bis(C₁-C₄alkyl)amino or cyclopropyl or by -COOC₁-C₃alkyl; or in which R₅ is C₃-C₄alkenyl or C₃-C₄alkynyl, or a phenyl group which is unsubstituted or substituted by halogen, methyl or methoxy, or an acyl radical of the meaning g); and R₆ is hydrogen or a C₁-C₄alkyl group, which is unsubstituted or substituted by halogen, hydroxyl, C₁-C₄alkoxy, C₁-C₄alkylamino or bis(C₁-C₄alkyl)amino, or in which R₆ is C₃-C₄alkenyl or C₃-C₄alkynyl.

4. A compound according to claim 3, in which R₁ and R₂ are hydrogen, R₃ is cyclopropyl, R₄ is halogen, -OR₅ or -NR₅R₆ and R₅ is hydrogen or R₆, and R₆ is C₁-C₃alkyl, which is unsubstituted or monosubstituted by C₁-C₄alkoxy or cyclopropyl or substituted by up to 5 halogen atoms; or C₃-C₄alkenyl or alkynyl.

5. A compound according to claim 1, in which R₁ and R₂ are hydrogen; and R₃ is C₃-C₄cycloalkyl, which is unsubstituted or monosubstituted by methyl or halogen; and R₄ is halogen.

6. A process for the preparation of a compound of the formula I, which comprises
a) reacting a phenylguanidinium salt of the formula IIa 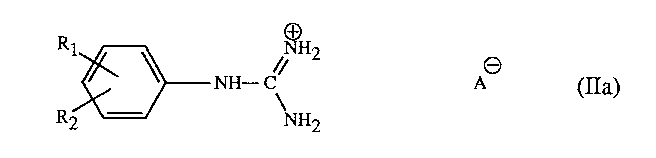 in which A^{⊖} is any desired anion, or the guanidine of the formula IIb on which the salt is based 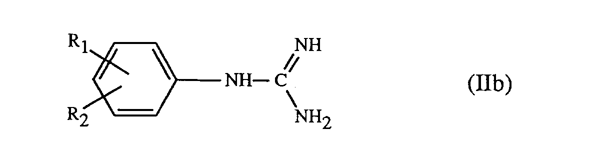 with a β-keto ester of the formula III 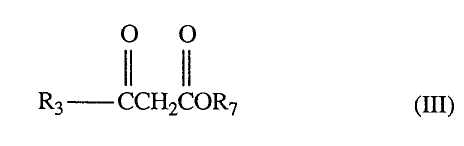 in which R₁, R₂ and R₃ are as defined for formula I and R₇ is C₃-C₆alkenyl, C₃-C₆alkinyl, benzyl which is unsubstituted or substituted by halogen and/or C₁-C₃alkyl or C₁-C₈alkyl, without a solvent or in a solvent, at temperatures of 60°C to 160°C, to give the compound Ia; and if desired acylating, carbamoylating or carbonylating the OH group, or replacing this group by halogen to give a compound of the formula Ib, and, if desired, replacing the halogen atom further by one of the other groups mentioned for R₄ 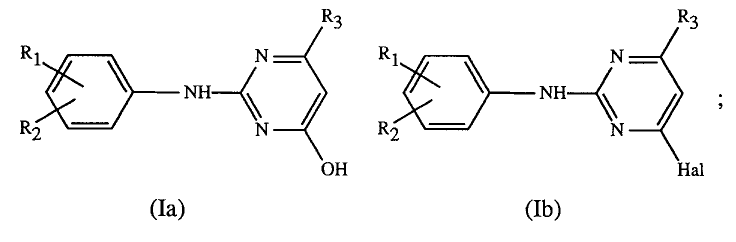 or
b) reacting an isothiuronium salt of the formula IV 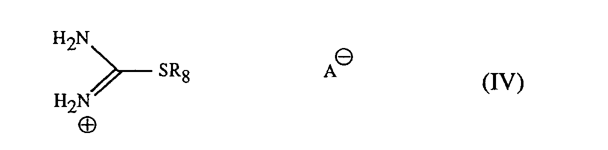 in which A^{⊖} is any desired anion, and R₈ is C₁-C₈alkyl, or benzyl which is unsubstituted or substituted by halogen and/or C₁-C₄alkyl, with a β-keto ester of formula III, preferably in a solvent, at temperatures from 40°C to 140°C, the thiopyrimidine V being formed, 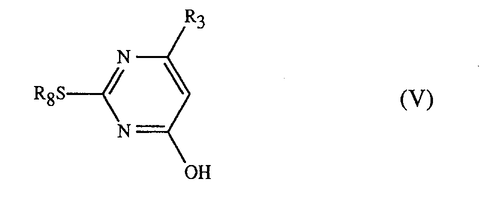 which is oxidized with an oxidizing agent to give the pyrimidine compound of the formula VI 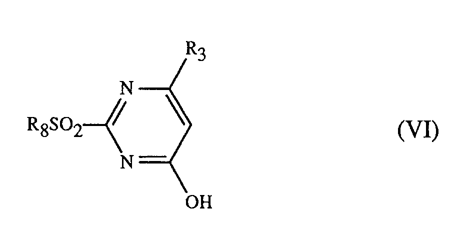 and this is reacted with a formylaniline of the formula VII 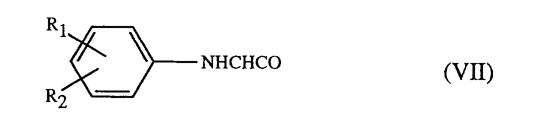 in an inert solvent in the presence of a base at temperatures between -30°C and +120°C to give a compound of the formula VIII 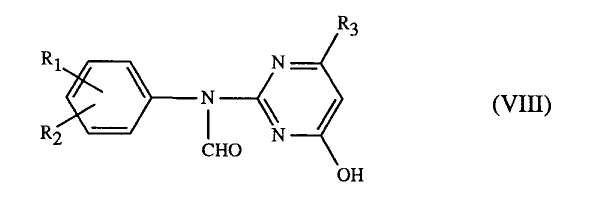 after which the resulting compound VIII is subjected to hydrolysis in the presence of a base or an acid at temperatures of 10°C to 110°C, to give compounds of the formula Ia, and, if desired, replacing the OH group by halogen to obtain compounds of the formula Ib and, if desired, further replacing the halogen by one of the other groups mentioned for R₄.

7. A microbicidal composition comprising, besides customary carrier material, a compound of the formula I according to any one of claims 1-5 as the active component.

8. A process for combating or preventing attack of crop plants by phytopathogenic microorganisms, wherein a compound of the formula I according to any one of claims 1-5 is applied to the plants, to parts of the plants or to their location as the active compound.

## Claims (Claims for the following Contracting State(s): ES)

1. A process for the preparation of a compound of the formula I 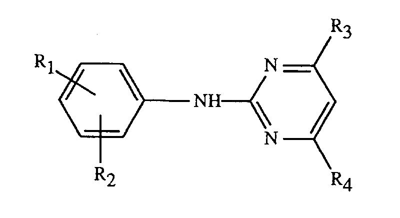 in which: R₁ and R₂ are hydrogen; R₃ is C₃-C₆cycloalkyl or C₃-C₆cycloalkyl which is substituted by up to three identical or different methyl or halogen groups; R₄ is halogen, thiocyano(-SCN), -OR₅, -SR₅ or -NR₅R₆, in which R₅
a) - is hydrogen, unsubstituted C₁-C₈alkyl or a C₁-C₄alkyl group which is substituted by halogen, hydroxyl, cyano, C₁-C₄alkoxy, C₁-C₄alkylamino, bis(C₁-C₄alkyl)amino or C₃-C₆cycloalkyl or by substituted or unsubstituted phenyl or by -CO-OC₁-C₃alkyl; or
b) - is C₃-C₆cycloalkyl which is unsubstituted or substituted by methyl; or
c) - is C₃-C₆alkenyl which is unsubstituted or substituted by halogen; or
d) - is C₃-C₆alkynyl which is unsubstituted or substituted by halogen; or
e) - is phenyl which is unsubstituted or substituted by halogen, C₁-C₃alkyl, C₁-C₃alkoxy or nitro; or
f) - is a 5-membered or 6-membered heterocyclic radical which is bonded via -CH₂- if appropriate, contains one to three hetero atoms N, O or S and is unsubstituted or substituted by halogen, C₁-C₃alkyl, C₁-C₃alkoxy or nitro; or
g) - is an acyl radical -CO-R', in which R' is C₁-C₆alkyl which is unsubstituted or substituted by halogen or C₁-C₃alkoxy; C₃-C₆alkenyl which is unsubstituted or substituted by halogen; or phenyl which is unsubstituted or substituted by halogen, C₁-C₃alkyl, C₁-C₃alkoxy or nitro; or
h) - is a carbamoyl radical -CO-NH-R'' or an oxycarbonyl radical -CO-OR'', in which R'' is an aliphatic or cycloaliphatic radical having not more than 6 C atoms, which is unsubstituted or halogen-substituted, or in which R'' is a phenyl or benzyl radical, which is in each case unsubstituted or substituted in the aromatic ring by halogen, C₁-C₃alkyl, C₁-C₃alkoxy or nitro; and in which
R₆ is hydrogen, unsubstituted C₁-C₈alkyl or C₁-C₄alkyl which is substituted by halogen, hydroxyl, cyano or C₁-C₄alkoxy; or in which R₆ is C₃-C₆cycloalkyl which is unsubstituted or substituted by methyl; C₃-C₆alkenyl or C₃-C₆alkynyl which is in each case unsubstituted or substituted by halogen; or phenyl which is unsubstituted or substituted by halogen, C₁-C₃alkyl, C₁-C₃alkoxy or nitro; and wherein, in the case where R₄ = NR₅R₆, the substituents R₅ and R₆, together with the N atom, can also together form an aziridine ring or a 5- or 6-membered heterocyclic radical, which can also contain one or two hetero atoms N, O or S in addition to the N atom and can be alkyl-substituted, which comprises
a) reacting a phenylguanidinium salt of the formula IIa 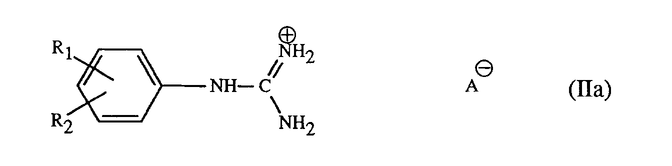 in which A^{⊖} is any desired anion, or the guanidine of the formula IIb on which the salt is based 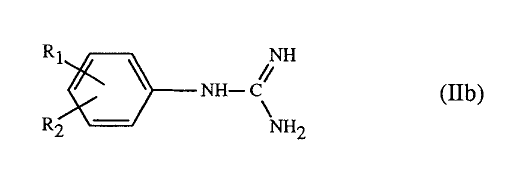 with a β-keto ester of the formula III 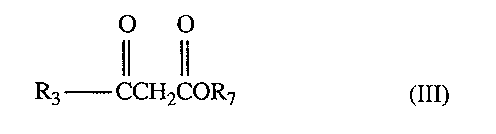 in which R₁, R₂ and R₃ are as defined for formula I and R₇ is C₃-C₆alkenyl, C₃-C₆alkinyl, benzyl which is unsubstituted or substituted by halogen and/or C₁-C₃alkyl or C₁-C₈alkyl, without a solvent or in a solvent, at temperatures of 60°C to 160°C, to give the compound Ia; and if desired acylating, carbamoylating or carbonylating the OH group, or replacing this group by halogen to give a compound of the formula Ib, and, if desired, replacing the halogen atom further by one of the other groups mentioned for R₄ 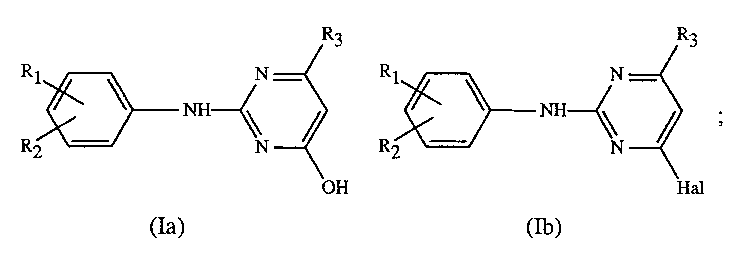 or
b) reacting an isothiuronium salt of the formula IV 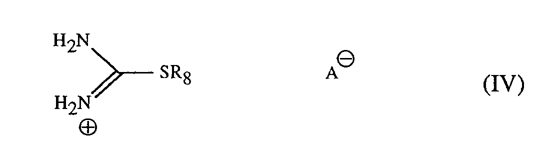 in which A^{⊖} is any desired anion, and R₈ is C₁-C₈alkyl, or benzyl which is unsubstituted or substituted by halogen and/or C₁-C₄alkyl, with a β-keto ester of formula III, preferably in a solvent, at temperatures from 40°C to 140°C, the thiopyrimidine V being formed, 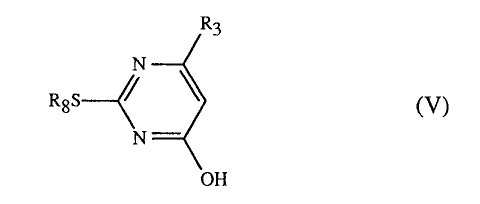 which is oxidized with an oxidizing agent to give the pyrimidine compound of the formula VI 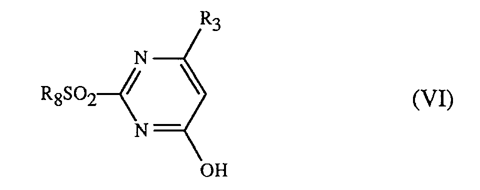 and this is reacted with a formylaniline of the formula VII 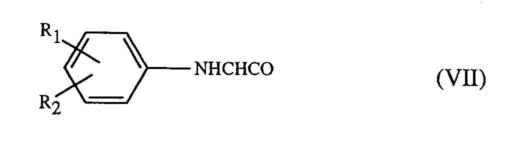 in an inert solvent in the presence of a base at temperatures between -30°C and +120°C to give a compound of the formula VIII 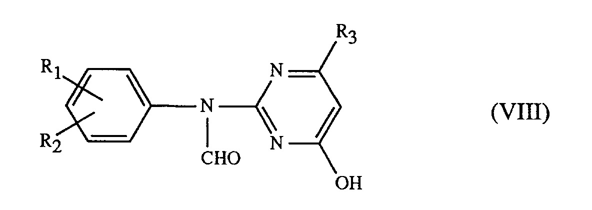 after which the resulting compound VIII is subjected to hydrolysis in the presence of a base or an acid at temperatures of 10°C to 110°C, to give compounds of the formula Ia, and, if desired, replacing the OH group by halogen to obtain compounds of the formula Ib and, if desired, further replacing the halogen by one of the other groups mentioned for R₄.

2. A process according to claim 1, in which R₁ and R₂ are hydrogen; and R₃ is C₃-C₄cycloalkyl, which is unsubstituted or monosubstituted by methyl or halogen; and R₄ is halogen, -OR₅ or -NR₅R₆, in which R₅ has the meanings a), b), c), d) or g) and R₆ is hydrogen or an alkyl, alkenyl or alkynyl radical, which can be unsubstituted or halogen-substituted if appropriate; or in which R₅ and R₆, together with the common N atom, are an aziridine ring or a piperidine or morpholine ring which is unsubstituted or methyl-substituted if appropriate.

3. A process according to claim 1, in which: R₁ and R₂ are hydrogen; R₃ is cyclopropyl or cyclopropyl which is substituted by one or two identical or different methyl or halogen groups; R₄ is halogen, -SR₅, -OR₅ or -NR₅R₆; and R₅ is hydrogen or a C₁-C₄alkyl group, which is unsubstituted or substituted by halogen, hydroxyl, C₁-C₄alkoxy, C₁-C₄alkylamino, bis(C₁-C₄alkyl)amino or cyclopropyl or by -COOC₁-C₃alkyl; or in which R₅ is C₃-C₄alkenyl or C₃-C₄alkinyl, or a phenyl group which is unsubstituted or substituted by halogen, methyl or methoxy, or an acyl radical of the meaning g); and R₆ is hydrogen or a C₁-C₄alkyl group, which is unsubstituted or substituted by halogen, hydroxyl, C₁-C₄alkoxy, C₁-C₄alkylamino or bis(C₁-C₄alkyl)amino, or in which R₆ is C₃-C₄alkenyl or C₃-C₄alkynyl.

4. A process according to claim 3, in which R₁ and R₂ are hydrogen, R₃ is cyclopropyl, R₄ is halogen, -OR₅ or -NR₅R₆ and R₅ is hydrogen or R₆, and R₆ is C₁-C₃alkyl, which is unsubstituted or monosubstituted by C₁-C₄alkoxy or cyclopropyl or substituted by up to 5 halogen atoms; or C₃-C₄alkenyl or alkynyl.

5. A process according to claim 1, in which R₁ and R₂ are hydrogen; and R₃ is C₃-C₄cycloalkyl, which is unsubstituted or monosubstituted by methyl or halogen; and R₄ is halogen.

## Revendications (Revendications pour l'(les) Etat(s) contractant(s) suivant(s): CH, DE, DK, FR, GB, IT, LI, NL)

1. Composés de formule I 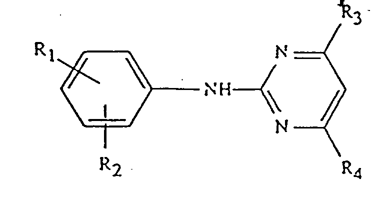 dans laquelle
R₁ et R₂ représentent l'hydrogène,
R₃ représente un groupe cycloalkyle en C3-C6 ou un groupe cycloalkyle en C3-C6 portant jusqu'à trois substituants identiques ou différents choisis parmi les groupes méthyle et les halogènes;
R₄ représente un halogène, un groupe thiocyano (-SCN), -OR₅, -SR₅, -NR₅R₆ dans lesquels
R₅ représente
a) l'hydrogène, un groupe alkyle en C1-C8 non substitué ou un groupe alkyle en C1-C4 substitué par des halogènes, des groupes hydroxy, cyano, alcoxy en C1-C4, alkylamino en C1-C4, bis-(alkyle en C1-C4)amino, cycloalkyle en C3-C6 ou par un groupe phényle lui-même substitué ou non, ou par un groupe -CO-O-alkyle en C1-C3; ou bien
b) un groupe cycloalkyle en C3-C6 non substitué ou substitué par un groupe méthyle; ou bien
c) un groupe alcényle en C3-C6 non substitué ou substitué par un halogène; ou bien
d) un groupe alcynyle en C3-C6 non substitué ou substitué par un halogène; ou bien
e) un groupe phényle non substitué ou substitué par des halogènes, des groupes alkyle en C1-C3, alcoxy en C1-C3, nitro; ou bien
f) un radical hétérocyclique à cinq ou six chaînons, éventuellement relié par l'intermédiaire d'un groupe -CH₂-, contenant un à trois hétéroatomes N, O ou S, non substitué ou substitué par des halogènes, des groupes alkyle en C1-C3, alcoxy en C1-C3, nitro; ou bien
g) un groupe acyle -CO-R' dans lequel R' représente un groupe alkyle en C1-C6 non substitué ou substitué par des halogènes, des groupes alcoxy en C1-C3; un groupe alcényle en C3-C6 non substitué ou substitué par des halogènes; un groupe phényle non substitué ou substitué par des halogènes, des groupes alkyle en C1-C3, alcoxy en C1-C3, nitro; ou bien
h) un groupe carbamoyle -CO-NH-R'' ou un groupe oxycarbonyle -CO-OR'' dans lesquels R'' représente un radical aliphatique ou cycloaliphatique à six atomes de carbone au maximum, qui est non substitué ou substitué par des halogènes, ou bien R'' représente un groupe phényle ou benzyle, chacun d'eux non substitué ou substitué dans le noyau aromatique par des halogènes, des groupes alkyle en C1-C3, alcoxy en C1-C3, nitro; et
R₆ représente l'hydrogène, un groupe alkyle en C1-C8 non substitué ou un groupe alkyle en C1-C4 substitué par des halogènes, des groupes hydroxy, cyano, alcoxy en C1-C4; ou bien R₆ représente un groupe cycloalkyle en C3-C6 non substitué ou substitué par des groupes méthyle; un groupe alcényle en C3-C6 ou alcynyle en C3-C6, chacun d'eux non substitué ou substitué par des halogènes; un groupe phényle non substitué ou substitué par des halogènes, des groupes alkyle en C1-C3, alcoxy en C1-C3, nitro; et dans le cas où R₄ = NR₅R₆, les substituants R₅ et R₆ peuvent également former ensemble et avec l'atome d'azote un cycle aziridine ou un radical hétérocyclique à cinq ou six chaînons qui, en plus de l'atome d'azote, peut encore contenir un ou deux hétéroatomes N, O ou S et peut être substitué par des groupes alkyle.

2. Composés selon revendication 1, pour lesquels R₁ et R₂ représentent l'hydrogène et R₃ représente un cycle cycloalkyle en C3-C4 non substitué ou monosubstitué par un groupe méthyle ou par un halogène;
R₄ représente un halogène, un groupe -OR₅, -NR₅R₆ dans lesquels
R₅ a les significations indiquées sous a), b), c), d) ou g) et
R₆ représente l'hydrogène ou un groupe alkyle, alcényle ou alcynyle, non substitué ou substitué par des halogènes; ou bien
R₅ et R₆ forment ensemble et avec l'azote commun un cycle aziridine ou un cycle pipéridine ou morpholine non substitué ou substitué par un groupe méthyle.

3. Composés selon revendication 1, pour lesquels :
R₁ et R₂ représentent l'hydrogène,
R₃ représente un groupe cyclopropyle ou un groupe cyclopropyle portant un ou deux substituants identiques ou différents choisis parmi les groupes méthyle et les halogènes;
R₄ représente un halogène, un groupe -SR₅, -OR₅, -NR₅R₆; et
R₅ représente l'hydrogène ou un groupe alkyle en C1-C4 non substitué ou substitué par des halogènes, des groupes hydroxy, alcoxy en C1-C4, alkylamino en C1-C4, bis(alkyle en C1-C4)amino, cyclopropyle ou -COO-alkyle en C1-C3; ou bien R₅ représente un groupe alcényle en C3-C4 ou alcynyle en C3-C4 ou bien un groupe phényle non substitué ou substitué par des halogènes, des groupes méthyle ou méthoxy, ou un groupe acyle selon la signification g); et R₆ représente l'hydrogène ou un groupe alkyle en C1-C4 non substitué ou substitué par des halogènes, des groupes hydroxy, alcoxy en C1-C4, alkylamino en C1-C4 ou bis-(alkyle en C1-C4)amino-, ou bien R₆ représente un groupe alcényle en C3-C4 ou alcynyle en C3-C4.

4. Composés selon revendication 3 pour lesquels
R₁ et R₂ représentent l'hydrogène,
R₃ représente un groupe cyclopropyle,
R₄ représente un halogène, un groupe -OR₅ ou -NR₅R₆ et
R₅ représente l'hydrogène ou a la même signification que R₆ et
R₆ représente un groupe alkyle en C1-C3 non substitué ou portant un substituant alcoxy en C1-C4 ou cyclopropyle, ou bien jusqu'à cinq substituants halogéno; ou bien un groupe alcényle en C3-C4 ou alcynyle.

5. Composés selon revendication 1, pour lesquels R₁ et R₂ représentent l'hydrogène et R₃ représente un groupe cycloalkyle en C3-C4 non substitué ou portant un substituant méthyle ou halogéno et
R₄ représente un halogène.

6. Procédé de préparation des composés de formule I, caractérisé en ce que
a) on fait réagir un sel de phénylguanidine de formule IIa 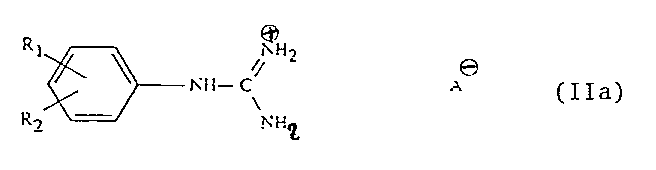 dans laquelle A^{⊖} représente un anion quelconque, ou bien la guanidine libre de formule IIb 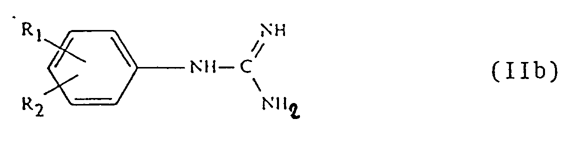 avec un bêta-cétoester de formule III 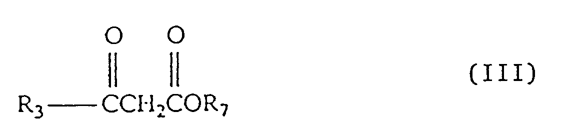 R₁, R₂, R₃ ayant les significations indiquées en référence à la formule I et R₇ représentant un groupe alcényle en C3-C6, alcynyle en C3-C6, benzyle non substitué ou substitué par des halogènes et/ou des groupes alkyle en C1-C3, ou un groupe alkyle en C1-C8, en l'absence de solvant ou dans un solvant, à des températures de 60 à 160°C, la réaction donnant le composé Ia; et si on le désire, on acyle, on carbamoyle ou on carbonyle le groupe OH, ou bien on l'échange contre un halogène afin d'obtenir les composés de formule Ib; et si on le désire, on réalise d'autres échanges de l'atome d'halogène contre l'un des autres groupes mentionnés en référence à R₄ 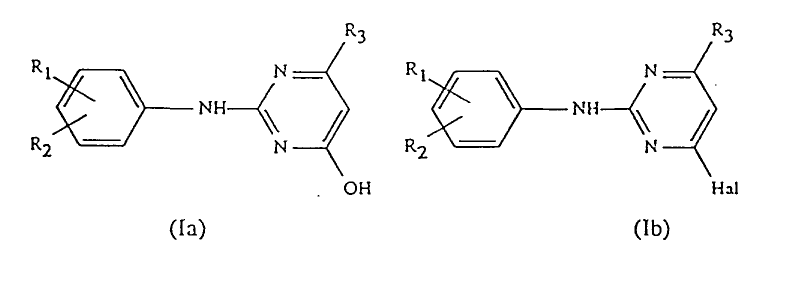 ou bien
b) on fait réagir un sel d'isothiuronium de formule IV 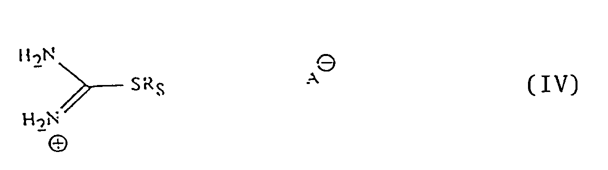 dans laquelle A^{⊖} représente un anion quelconque et R₈ représente un groupe alkyle en C1-C8 ou bien un groupe benzyle non substitué ou substitué par des halogènes et/ou des groupes alkyle en C1-C4, avec un bêta-cétoester de formule III, de préférence dans un solvant, à des températures de 40 à 140°C, ce qui donne la thiopyrimidine V 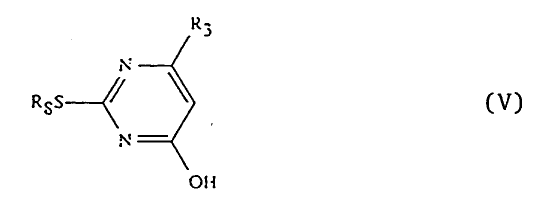 qu'on oxyde à l'aide d'un agent oxydant en le dérivé de pyrimidine de formule VI 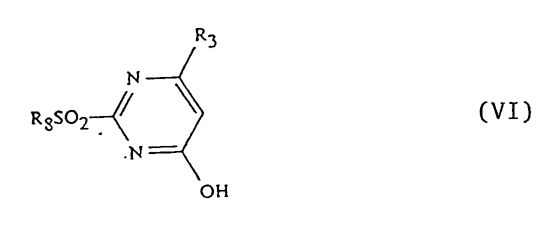 qu'on fait réagir avec une formylaniline de formule VII 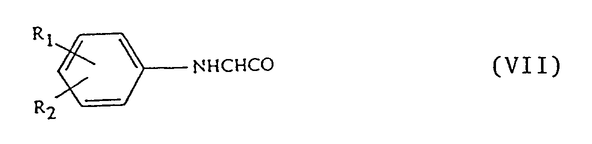 dans un solvant inerte en présence d'une base à des températures de -30 à +120°C, ce qui donne un composé de formule VIII 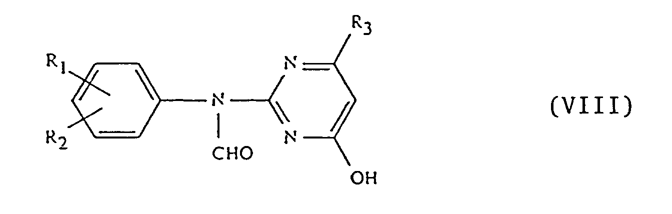 après quoi, on soumet ce composé VIII à une hydrolyse en présence d'une base ou d'un acide à des températures de 10 à 110°C, ce qui donne des composés de formule Ia et, si on le désire, on échange le groupe OH contre un halogène, ce qui donne des composés de formule Ib et, si on le désire, on procède à d'autres échanges de l'halogène contre l'un des autres groupes mentionnés en référence à R₄.

7. Produit microbicide contenant en tant que composant actif, avec des véhicules usuels, un composé de formule I selon l'une des revendications 1 à 5.

8. Procédé pour combattre ou prévenir une attaque de végétaux cultivés par des microorganismes phytopathogènes, caractérisé en ce que l'on applique sur la plante, des parties de la plante ou l'habitat de la plante, une substance active consistant en un composé de formule I selon l'une des revendications 1 à 5.

## Revendications (Revendications pour l'(les) Etat(s) contractant(s) suivant(s): ES)

1. Procédé de préparation des composés de formule I 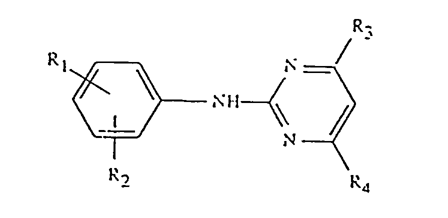 dans laquelle
R₁ et R₂ représentent l'hydrogène,
R₃ représente un groupe cycloalkyle en C3-C6 ou un groupe cycloalkyle en C3-C6 portant jusqu'à trois substituants identiques ou différents choisis parmi les groupes méthyle et les halogènes;
R₄ représente un halogène, un groupe thiocyano (-SCN), -OR₅, -SR₅, -NR₅R₆ dans lesquels
R₅ représente
a) l'hydrogène, un groupe alkyle en C1-C8 non substitué ou un groupe alkyle en C1-C4 substitué par des halogènes, des groupes hydroxy, cyano, alcoxy en C1-C4, alkylamino en C1-C4, bis-(alkyle en C1-C4)amino, cycloalkyle en C3-C6 ou par un groupe phényle lui-même substitué ou non, ou par un groupe -CO-O-alkyle en C1-C3; ou bien
b) un groupe cycloalkyle en C3-C6 non substitué ou substitué par un groupe méthyle; ou bien
c) un groupe alcényle en C3-C6 non substitué ou substitué par un halogène; ou bien
d) un groupe alcynyle en C3-C6 non substitué ou substitué par un halogène; ou bien
e) un groupe phényle non substitué ou substitué par des halogènes, des groupes alkyle en C1-C3, alcoxy en C1-C3, nitro; ou bien
f) un radical hétérocyclique à cinq ou six chaînons, éventuellement relié par l'intermédiaire d'un groupe -CH₂-, contenant un à trois hétéroatomes N, O ou S, non substitué ou substitué par des halogènes, des groupes alkyle en C1-C3, alcoxy en C1-C3, nitro; ou bien
g) un groupe acyle -CO-R' dans lequel R' représente un groupe alkyle en C1-C6 non substitué ou substitué par des halogènes, des groupes alcoxy en C1-C3; un groupe alcényle en C3-C6 non substitué ou substitué par des halogènes; un groupe phényle non substitué ou substitué par des halogènes, des groupes alkyle en C1-C3, alcoxy en C1-C3, nitro; ou bien
h) un groupe carbamoyle -CO-NH-R'' ou un groupe oxycarbonyle -CO-OR'' dans lesquels R'' représente un radical aliphatique ou cycloaliphatique à six atomes de carbone au maximum, qui est non substitué ou substitué par des halogènes, ou bien R'' représente un groupe phényle ou benzyle, chacun d'eux non substitué ou substitué dans le noyau aromatique par des halogènes, des groupes alkyle en C1-C3, alcoxy en C1-C3, nitro; et
R₆ représente l'hydrogène, un groupe alkyle en C1-C8 non substitué ou un groupe alkyle en C1-C4 substitué par des halogènes, des groupes hydroxy, cyano, alcoxy en C1-C4; ou bien R₆ représente un groupe cycloalkyle en C3-C6 non substitué ou substitué par des groupes méthyle; un groupe alcényle en C3-C6 ou alcynyle en C3-C6, chacun d'eux non substitué ou substitué par des halogènes; un groupe phényle non substitué ou substitué par des halogènes, des groupes alkyle en C1-C3, alcoxy en C1-C3, nitro; et dans le cas où R₄ = NR₅R₆, les substituants R₅ et R₆ peuvent également former ensemble et avec l'atome d'azote un cycle aziridine ou un radical hétérocyclique à cinq ou six chaînons qui, en plus de l'atome d'azote, peut encore contenir un ou deux hétéroatomes N, O ou S et peut être substitué par des groupes alkyle, caractérisé en ce que
a) on fait réagir un sel de phénylguanidine de formule IIa 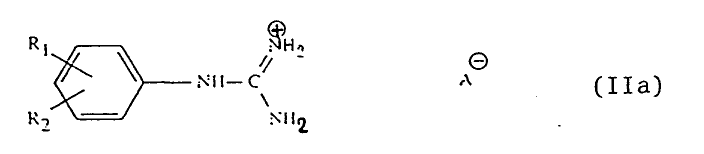 dans laquelle A^{⊖} représente un anion quelconque, ou bien la guanidine libre de formule IIb 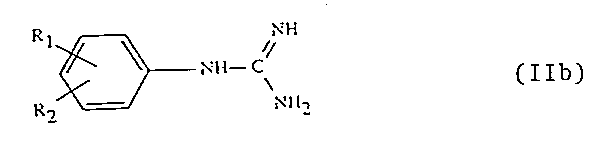 avec un bêta-cétoester de formule III 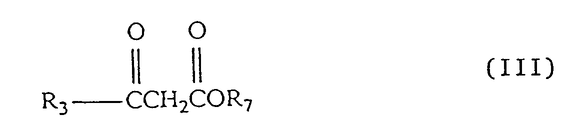 R₁, R₂, R₃ ayant les significations indiquées en référence à la formule I et R₇ représentant un groupe alcényle en C3-C6, alcynyle en C3-C6, benzyle non substitué ou substitué par des halogènes et/ou des groupes alkyle en C1-C3, ou un groupe alkyle en C1-C8, en l'absence de solvant ou dans un solvant, à des températures de 60 à 160°C, la réaction donnant le composé Ia; et si on le désire, on acyle, on carbamoyle ou on carbonyle le groupe OH, ou bien on l'échange contre un halogène afin d'obtenir les composés de formule Ib; et si on le désire, on réalise d'autres échanges de l'atome d'halogène contre l'un des autres groupes mentionnés en référence à R₄ 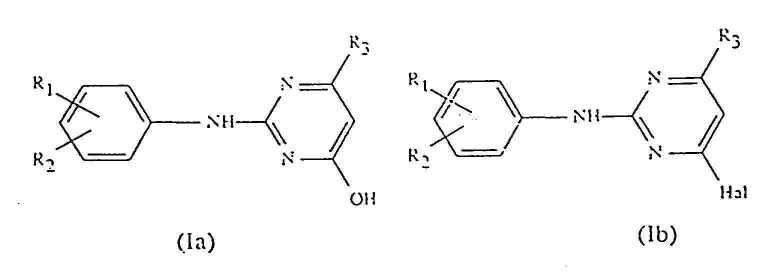 ou bien
b) on fait réagir un sel d'isothiuronium de formule IV 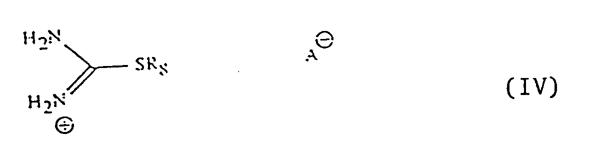 dans laquelle A^{⊖} représente un anion quelconque et R₈ représente un groupe alkyle en C1-C8 ou bien un groupe benzyle non substitué ou substitué par des halogènes et/ou des groupes alkyle en C1-C4, avec un bêta-cétoester de formule III, de préférence dans un solvant, à des températures de 40 à 140°C, ce qui donne la thiopyrimidine V 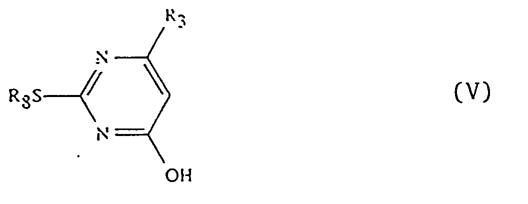 qu'on oxyde à l'aide d'un agent oxydant en le dérivé de pyrimidine de formule VI 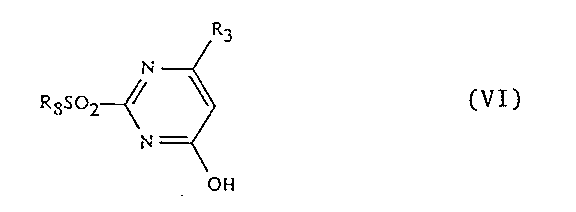 qu'on fait réagir avec une formylaniline de formule VII 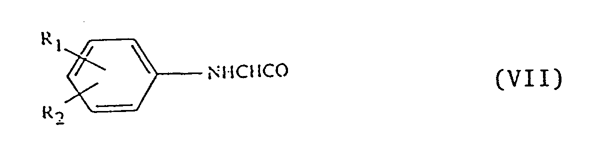 dans un solvant inerte en présence d'une base à des températures de -30 à +120°C, ce qui donne un composé de formule VIII 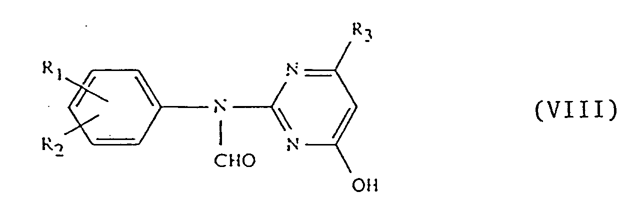 après quoi, on soumet ce composé VIII à une hydrolyse en présence d'une base ou d'un acide à des températures de 10 à 110°C, ce qui donne des composés de formule Ia et, si on le désire, on échange le groupe OH contre un halogène, ce qui donne des composés de formule Ib et, si on le désire, on procède à d'autres échanges de l'halogène contre l'un des autres groupes mentionnés en référence à R₄.

2. Procédé selon revendication 1, dans lequel
R₁ et R₂ représentent l'hydrogène et R₃ représente un cycle cycloalkyle en C3-C4 non substitué ou monosubstitué par un groupe méthyle ou par un halogène;
R₄ représente un halogène, un groupe -OR₅, -NR₅R₆ dans lesquels
R₅ a les significations indiquées sous a), b), c), d) ou g) et
R₆ représente l'hydrogène ou un groupe alkyle, alcényle ou alcynyle, non substitué ou substitué par des halogènes; ou bien
R₅ et R₆ forment ensemble et avec l'azote commun un cycle aziridine ou un cycle pipéridine ou morpholine non substitué ou substitué par un groupe méthyle.

3. Procédé selon revendication 1, dans lequel
R₁ et R₂ représentent l'hydrogène,
R₃ représente un groupe cyclopropyle ou un groupe cyclopropyle portant un ou deux substituants identiques ou différents choisis parmi les groupes méthyle et les halogènes;
R₄ représente un halogène, un groupe -SR₅, -OR₅, -NR₅R₆; et
R₅ représente l'hydrogène ou un groupe alkyle en C1-C4 non substitué ou substitué par des halogènes, des groupes hydroxy, alcoxy en C1-C4, alkylamino en C1-C4, bis(alkyle en C1-C4)amino, cyclopropyle ou -COO-alkyle en C1-C3; ou bien R₅ représente un groupe alcényle en C3-C4 ou alcynyle en C3-C4 ou bien un groupe phényle non substitué ou substitué par des halogènes, des groupes méthyle ou méthoxy, ou un groupe acyle selon la signification g); et R₆ représente l'hydrogène ou un groupe alkyle en C1-C4 non substitué ou substitué par des halogènes, des groupes hydroxy, alcoxy en C1-C4, alkylamino en C1-C4 ou bis-(alkyle en C1-C4)amino-, ou bien R₆ représente un groupe alcényle en C3-C4 ou alcynyle en C3-C4.

4. Procédé selon revendication 3, dans lequel
R₁ et R₂ représentent l'hydrogène,
R₃ représente un groupe cyclopropyle,
R₄ représente un halogène, un groupe -OR₅ ou -NR₅R₆, et
R₅ représente l'hydrogène ou a la même signification que R₆, et
R₆ représente un groupe alkyle en C1-C3 non substitué ou portant un substituant alcoxy en C1-C4 ou cyclopropyle, ou bien jusqu'à cinq substituants halogéno; ou bien un groupe alcényle en C3-C4 ou alcynyle.

5. Procédé selon revendication 1, dans lequel
R₁ et R₂ représentent l'hydrogène et R₃ représente un groupe cycloalkyle en C3-C4 non substitué ou portant un substituant méthyle ou halogéno et
R₄ représente un halogène.
